(19) Europäisches Patentamt
European Patent Office
Office européen des brevets

(11) **EP 3 658 050 B1**

(12) **EUROPEAN PATENT SPECIFICATION**

(45) Date of publication and mention
of the grant of the patent:
**06.09.2023 Bulletin 2023/36**

(51) International Patent Classification (IPC):
**A61B 18/14** (2006.01)        **A61B 18/00** (2006.01)

(21) Application number: **18752403.8**

(52) Cooperative Patent Classification (CPC):
**A61B 18/1492;** A61B 2018/00029;
A61B 2018/00238; A61B 2018/00357;
A61B 2018/00577; A61B 2018/00642;
A61B 2018/00702; A61B 2018/00761;
A61B 2018/00797; A61B 2018/00827;
A61B 2018/00875; A61B 2018/1467;
A61B 2218/002

(22) Date of filing: **24.07.2018**

(86) International application number:
**PCT/US2018/043563**

(87) International publication number:
**WO 2019/023280 (31.01.2019 Gazette 2019/05)**

(54) **ABLATION CATHETERS**

ABLATIONSKATHETER

CATHÉTERS D'ABLATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.07.2017 US 201762536855 P**

(43) Date of publication of application:
**03.06.2020 Bulletin 2020/23**

(73) Proprietor: **Affera, Inc.
Watertown, MA 02472 (US)**

(72) Inventors:
• **HARLEV, Doron
Watertown, MA 02472 (US)**
• **COLLIER, Ian, Matthew
Watertown, MA 02472 (US)**
• **WALLACE, Andrew, Miles
Watertown, MA 02472 (US)**

(74) Representative: **Zimmermann & Partner
Patentanwälte mbB
Postfach 330 920
80069 München (DE)**

(56) References cited:
EP-A1- 3 178 431        EP-A2- 3 238 646
US-A1- 2014 081 111        US-A1- 2016 051 321

**Description**

CROSS-REFERENCE TO RELATED APPLICATION(S)

The present application claims priority to U.S. Provisional Patent Application No. 62/536,855, filed July 25, 2017.

TECHNICAL FIELD

The present technology is generally related to ablation catheters and related systems and methods, whereby systems and methods are not covered by the claimed invention.

BACKGROUND

Abnormal rhythms, generally referred to as arrhythmia, can occur in the heart. Cardiac arrhythmias develop when abnormal conduction in the myocardial tissue modifies the typical heartbeat pattern. Radio frequency ("RF") catheter ablation can be used to form lesions that interrupt the mechanism of abnormal conduction to terminate certain arrhythmias.

Conventional ablation devices have narrow and small ablation electrodes that create small lesions in cardiac tissue. The ablation electrode(s) is on a distal end of a catheter shaft. The distal end with the ablation electrode(s) is narrow so that the distal end can pass through a narrow sheath used to deliver and position the ablation electrode(s) at the heart. Because of the small lesions created by the ablation electrode(s), the ablation electrode(s) is/are repeatedly positioned against a new region of the cardiac tissue and activated to ablate the tissue. These steps are repeated, as many times as necessary, in an effort to achieve the desired result. Current small area ablation electrodes can require 10 to 50 or more ablations to create a sufficient lesion pattern. In addition, the narrow ablation electrode(s) of conventional cardiac ablation catheters have a risk of unintentionally perforating or damaging cardiac tissue. In view of this risk, the force applied to the cardiac ablation catheter to push the ablation catheters against heart tissue is closely monitored and maintained below a threshold force or pressure.

European patent application no. EP3178431 A1 discloses an apparatus with an expandable structure and one or more sets of electrodes disposed on the surface of the expandable structure. US patent application no. 2014/081111 A1 discloses an ablation electrode including one or more mapping electrodes deposited on an exterior surface thereof at a distal end of a map and ablate catheter. US patent application no. 2016/051321 A1 discloses an ablation catheter comprising an inflatable balloon.

BRIEF DESCRIPTION OF THE DRAWINGS

The disclosure will be understood more fully from the detailed description given below and from the accompanying drawings of various implementations of the disclosure. The drawings, however, should not be taken to limit the disclosure to the specific implementations, but are for explanation and understanding only.

Figure 1 schematically illustrates an ablation system configured in accordance with an implementation of the present technology during an ablation treatment.

Figure 2 is a perspective view of an ablation catheter of the ablation system outlined in Figure 1 with a catheter housing that can act as a handle and a close-up view of a catheter shaft and a balloon.

Figures 3 is a perspective close-up view of a balloon of the ablation catheter of Figure 2.

Figures 4 is an angled cross-sectional view of a balloon of the ablation catheter of Figure 2.

Figure 5 is an enlarged frontal view of a balloon of the ablation catheter of Figure 2.

Figure 6 shows a cross-sectional view of a section of the ablation catheter showing the orientation of a sensing electrode, an element imbedded within the body and the fluid-filled area within the balloon.

Figure 7 schematically illustrates a method of inserting the ablation catheter of Figure 2 into a patient.

Figure 8 is a simplified cross-sectional view of the balloon of the catheter of Figure 2 showing a change in a wall thickness along the balloon.

Figure 9 is a simplified representation of the expandable balloon of the ablation catheter of Figure 2 in lateral contact with tissue.

Figure 10 is a simplified representation of the expandable balloon of the ablation catheter of Figure 2 showing propagation of isothermal lines relative to tissue in lateral contact with the expandable balloon.

Figure 11 is a simplified representation of the expandable balloon of the ablation catheter of Figure 2 in axial contact with tissue.

Figure 12 simplified representation of the expandable balloon of the ablation catheter of Figure 2 showing propagation of isothermal lines relative to tissue in axial contact with the expandable balloon.

Figures 13A-C show an implementation in which the neck of the catheter is deformable in response to tissue contact at the balloon.

Figures 14A and 14B show a theoretical rendering of lesions formed by a conventional narrow-tipped ablation electrode versus larger lesions formed by an electrode located on the outer surface of a balloon.

Figures 15A and 15B show a theoretical rendering of conventional ablation treatment.

Figures 16A and 16B show a theoretical rendering of an ablation treatment with a larger ablation tip.

Figure 17 shows a partial cross-sectional view of a balloon of the ablation catheter of Figure 2, with the open, cross-sectional area showing the interior of a catheter shaft defining a fluid delivery lumen and containing a heating electrode, printed circuits, irrigative holes or apertures, ablation, detection and other electrodes.

DETAILED DESCRIPTION

A. Overview

The present disclosure is directed to an ablation catheter. For the sake of clarity of explanation, the devices of the present disclosure are described in the context of cardiac ablation procedures. However, unless otherwise specified or made clear from the context, the devices of the present technology can be utilized in additional medical procedures including, but not limited to, ablating tumors in cancer treatment and electro-surgery procedures in which tissue is cut and substantially simultaneously cauterized to avoid or minimize bleeding.

In several implementations of the present technology, an ablation catheter is placed into the vasculature of the patient depending on whether the left ventricle or atrium or right ventricle or atrium of the heart is to be treated with ablation therapy. Frequently an artery or vein in the groin such as one of the femoral access is selected for catheterization. Alternatively, sub-xyphoid access by the device can allow for epicardial ablation. The catheter is introduced through patient vasculature through a port or insertion sheath and is advanced in the direction of the vena cava or aorta, depending on whether the right or left side of the heart is to be catheterized. Once in the heart, the catheter position can be adjusted to access the appropriate atrium or ventricle targets for ablation.

The ablation catheter extends from a housing at the proximal end of the ablation catheter and that remains external to the patient. By manipulating controls at the housing, a medical practitioner, such as a surgeon, controls the position, orientation and operation of the distal end of the ablation catheter in the vasculature. The position and orientation of the distal end can be controlled by manipulation or steering controls at the housing. The housing can act as a handle to position and orient the ablation catheter and particularly the balloon with the conductive outer surface. By manipulating the handle, the balloon in a collapsed state enters the patient vasculature and is advanced until positioned near or in the heart. The handle and other controls associated with the housing can be used to retract the sheath to expand (also referred to as deploy) the balloon such that the conductive outer surface forms a large area configured to contact cardiac tissue.

According to one aspect of the present technology, an ablation catheter includes a large area ablation electrode that can create large and shallow lesions in heart tissue. The ablation catheter can, further or instead, include improvements in areas such as user or practitioner feedback and control, and overall device capabilities. Compared to conventional cardiac ablation catheters, the ablation catheters described herein have a large surface area ablation electrode that collapses to move through a catheter sheath.

Conventional ablation conductive outer surfaces are rigid and limited by the size of the sheath through which the ablation catheter is passed to introduce the ablation catheter into patient vasculature. Lesions formed by such ablation catheters are limited by the approximate surface area of the ablation electrode that is brought into contact with targeted tissue. The small surface area ablation electrodes also can concentrate current such that the ablation energy applied to the heart tissue is limited to avoid unsafe levels of current density being applied to the tissue.

The ablation electrodes disclosed herein include a conductive surface; formed by a unitary conductive outer layer on an outer surface of the distal section of the balloon (e.g., a conductive ink or a conductive surface treatment on an outer surface of at least the distal section of the balloon) or such as formed by a conductive material forming the balloon. The balloon provides an elastomeric support that, in an expanded state (e.g., in the absence of an externally applied force) can have a bulbous shape. As used herein, a bulbous shape shall be understood to include any one or more of various different shapes in which at least a distal section of the balloon is substantially hemispherical. Thus, for example, the bulbous shape of the balloon can include a substantially spherical shape. Additionally, or alternatively, the bulbous shape of the balloon can include a substantially hemispherical distal section and a substantially frustoconical proximal section. Such asymmetry of shape between the distal section and the proximal section of the balloon can be useful, for example, for achieving preferential deformation of the distal section of the balloon.

The balloon with the conductive outer surface is collapsible to fit in a sheath (e.g., an 8 Fr sheath, as is commonly used in cardiac procedures) during advancement to the heart. The balloon expands after extending beyond the sheath. The expansion allows outer conductive surface area on the balloon to be substantially increased, e.g., an increase of 50 to over 100 percent, wherein the outer conductive surface area is a bulbous shape, semi-spherical shape or other shape having a gently curved or curvilinear surface.

The large area of the conductive surface creates a large ablation electrode that can be used to form large area lesions in heart tissue and safely deliver high levels of electrical energy to form the lesions. Further, the expanded balloon can be hollow or porous such that a saline solution or other liquid can flow through the balloon, out of and over the outer conductive surface to provide irrigation of the contact area between the ablation electrode and heart tissue.

The balloon decouples the physical limitations associated with to the size of the ablation electrode and the size of a catheter sheath. The balloon is collapsible to move through the sheath. While collapsed, the conductive outer surface of the balloon can have overlapping folds and be substantially reduced in size. When the balloon is expanded, the conductive surface expands, such as by unfolding to have a large conductive surface area suitable for contact against cardiac tissue.

The expansion of the balloon and the outer conductive surface may not physically increase the surface area of either. The expansion can be similar in concept to the expansion of an umbrella when it is opened. Similarly, a metallic party balloon does not increase in surface area as it is inflated from a collapsed state to an inflated state. Nevertheless, the expansion could include stretching of the balloon if the material forming the balloon stretched under the pressure of the saline solution flowing through the balloon. If the balloon stretches, the outer conductive surface can be likewise stretchable to reduce the likelihood of breaking as the balloon expands. The conductive surface can have, for example, an embedded spiral wire that provides good conduction and allows for stretching of the surface.

The balloon can also, or instead, support other structures such as saline irrigation openings, external and internal sensing electrodes, thermal sensors and pressure sensors. The external sensing electrodes can each be a concentric pair of electrodes on the outer surface of the balloon. Several concentric pairs of electrodes can be distributed symmetrically over the outer surface of the balloon. Signals from the sensing electrodes, such as current, resistance and voltage signals, can be analyzed in real time or near real time to provide data to measure impedance, current, temperature, pressure, and other parameters. The signals can be at different frequencies. For example, the ablation energy can be applied at a frequency of 500 kHz (kilohertz) and signals at that frequency can be analyzed to determine characteristics of the application of the ablation energy. Signals in a 5 kHz to 10 kHz range can result from energy in that frequency range being applied to sensing electrodes and used to evaluate impedances and other characteristics between the sensing electrodes. Signals below 500 Hz can indicate one or more of electrical activity of the heart, movement of the heart, and respiration of the patient. These biological signals can also be analyzed. An analysis system can include a signal receiver that tunes to various frequencies to detect each type of signal. One or more of the signals can provide data that is analyzed by the analysis system to provide instantaneous feedback on important parameters including one or more of tissue contact by the ablation electrode, deformation of the balloon, and the progress of tissue ablation.

The catheter system can be associated with one or more computer systems that generate the ablation energy and analyze signals from the ablation electrode and sensing electrodes. The one or more computer systems can present results of the analyses of the impedance and other measurements. The results can report whether and the extent to which the balloon is in contact with heart tissue, whether ablation of tissue has been achieved, an indication as to the state of the resulting lesion progress, and/or combinations thereof.

Temperature sensors within the balloon and positioned near the outer conductive surface can provide data indicating the extent of contact between the conductive surface and heart tissue and, further or instead, the extent of heating of the heart tissue. The temperature and other factors at or near the surface of the tissue in contact with the conductive outer surface can be indicative of whether ablation has occurred and the depth of the ablation. Monitoring the temperature at the surface of the conductive outer surface or at more than one point on the tip can provide information about the state of the contacted tissue.

The ablation catheter can be connected to a generator of ablation energy; a pump providing saline to irrigate the balloon, and signal analyzers that process signals from the ablation electrode and sensing electrodes. Sensing can be performed by one or more surface electrodes disposed along the outer or inner surface of the balloon, which can be hollow to accept fluid from the delivery lumen and dimensioned to expand to a diameter greater than the catheter shaft. The generator of ablation energy can produce or apply radio frequency (RF) energy, electroporation, microwave energy, DC, cryoablation, and/or combinations thereof.

RF energy is generated by an external RF energy generator and conducted through the catheter to an ablation electrode at the distal end of the catheter. The RF energy is applied as high-frequency electrical currents from the ablation electrode to the heart tissue which heats the tissue. The heating results in selective ablation of the nearby tissue and the formation of non-conductive scar tissue.

According to another aspect of the present technology not covered by the claimed invention, a method of treating a heart in a mammalian patient includes advancing an ablation catheter through a vascular system of the patient and positioning in the heart a single ablation electrode, the single ablation electrode at a distal end portion of the ablation catheter, wherein the single ablation electrode includes an expandable structure with a conductive outer surface; during or after the advancement of the ablation catheter, expanding the expandable structure; positioning the conductive outer surface of the expanded balloon against heart tissue, and applying electromagnetic energy to the conductive outer surface to ablate the heart tissue.

According to still another aspect of the present technology, a catheter assembly includes a catheter configured for insertion into a vascular system of a patient; an expandable structure attached to a distal region of the catheter, and a pair of concentric sensing electrodes on an outer surface of the expandable structure.

According to yet another aspect of the present technology, an ablation catheter assembly includes an expandable structure formed of a deformable elastomeric material, wherein the balloon in an expanded state encompasses a cross sectional area greater than a cross sectional area of a catheter shaft to which the balloon is attached, and an ablation electrode comprising a conductive outer layer on an outer surface of the expandable structure, wherein the conductive outer surface is configured to abut tissue in the heart.

According to still another aspect of the present technology not covered by the claimed invention, a method of treating a heart in a mammalian patient includes advancing an ablation catheter through a vascular system of the patient and into the heart; positioning a non-conductive balloon at a distal end of the ablation catheter against cardiac tissue in the heart, wherein the balloon with a conductive outer surface configured to contact the cardiac tissue; applying electromagnetic energy to the conductive outer surface while the conductive outer surface is in contact with the cardiac tissue, measuring an electrical parameter based on electrical energy flowing through sensing electrodes on the balloon, wherein the sensing electrodes include sensing electrodes located in non-conductive islands of the conductive outer surface, and based on the measured electrical parameter, determining at least one of a characteristic of contact between the conductive outer surface and the heart tissue, and a characteristic of an ablation of the heart tissue at the contact with the conductive outer surface.

According to yet another aspect of the present technology, an ablation catheter includes a catheter shaft; a collapsible, hollow and non-conductive bulbous balloon secured to a distal end of the catheter shaft, and a conductive skin on an outer surface on the bulbous balloon, wherein the conductive surface is configured as a single ablation electrode.

According to still another aspect of the present technology, a catheter includes a catheter shaft, a balloon, an ablation electrode, and a plurality of sensing electrodes. The catheter shaft has a proximal end portion and a distal end portion, and the catheter shaft defines an irrigation lumen. The balloon includes a proximal section and a distal section, the proximal section of the balloon is coupled to the distal end portion of the catheter shaft, the balloon defines a volume in fluid communication with the irrigation lumen, and the balloon defines a plurality of irrigation orifices in fluid communication with the volume. The ablation electrode is disposed along at least the distal section of the balloon. The plurality of sensing electrodes is disposed along at least the distal section of the balloon, each sensing electrode electrically isolated from the ablation electrode and each sensing electrode bounded by the ablation electrode.

According to yet another aspect of the present technology, a catheter includes a catheter shaft, a balloon, a plurality of sensing electrodes, and an ablation electrode. The catheter shaft has a proximal end portion and a distal end portion, the proximal end portion and the distal end portion define a longitudinal axis, and the catheter shaft defines an irrigation lumen. The balloon defines a plurality of irrigation orifices in fluid communication with the irrigation lumen. The balloon includes a proximal section and a distal section, the proximal section of the balloon coupled to the distal end portion of the catheter shaft. The distal section of the balloon has a surface area along a volume of revolution of the longitudinal axis. The plurality of sensing electrodes is along the distal section of the balloon. The ablation electrode is along the distal section of the balloon, and a combination of the ablation electrode and the plurality of sensing electrodes span substantially the entire surface area (e.g., greater than about 95 percent) of the distal section of the balloon.

According to still another aspect of the present technology, a catheter includes a catheter shaft, a balloon, a plurality of sensing electrodes, and an ablation electrode. The catheter shaft defines an irrigation lumen. The balloon defines a plurality of irrigation orifices in fluid communication with the irrigation lumen. The balloon includes a proximal section and a distal section, and the proximal section of the balloon is coupled to the catheter shaft. The plurality of sensing electrodes is disposed along the distal section of the balloon, and the ablation electrode is disposed along the distal section of the balloon. Independent of an orientation of the distal section of the balloon to tissue, any line of contact between the distal section of the balloon and the tissue is substantially continuously spanned by the ablation electrode, one or more of the sensing electrodes, or a combination thereof.

In certain implementations, along an outer surface of the balloon, the ablation electrode can extend between each of the plurality of sensing electrodes and each of the other sensing electrodes of the plurality of sensing electrodes.

In some implementations, the volume defined by the balloon can be in thermal communication with the ablation electrode.

In certain implementations, at least the distal section of the balloon can be semi-compliant. For example, in response to an axial force, the distal section of the balloon can be more compliant than the proximal section of the balloon. Additionally, or alternatively, the balloon can include a neck disposed along the proximal section of the balloon. The neck can be, for example, self-expandable to expand the balloon from a collapsed state to an expanded state.

In some implementations, a thickness of the proximal section of the balloon can be greater than a thickness of the distal section of the balloon.

In certain implementations, at least some irrigation orifices of the plurality of irrigation orifices extend through the ablation electrode.

In some implementations, the plurality of irrigation orifices can be positioned along each of the distal section of the balloon and the proximal section of the balloon.

In certain implementations, at least some irrigation orifices of the plurality of irrigation orifices are positioned to direct fluid in a proximal direction relative to the balloon.

The ablation electrode includes a unitary conductive outer layer on an outer surface of the distal section of the balloon. For example, the unitary conductive outer layer can include a conductive ink disposed on the outer surface of the balloon.

In certain implementations, the balloon can be expandable (e.g., inflatable) from a collapsed state to an expanded state such as, for example, in the absence of an external force applied to an external surface of the balloon. In the collapsed state, the balloon can be deliverable through, for example, an 8 Fr sheath.

In some implementations, the catheter can further include a plurality of temperature sensors disposed along the balloon. Each temperature sensor can be thermally isolated from irrigation fluid in the volume defined by the balloon. Additionally, or alternatively, at least some of the plurality of temperature sensors are disposed along the distal section of the balloon.

In certain implementations, the catheter can further include flexible printed circuits disposed along the balloon, wherein the sensing electrodes are coupled to the respective flexible printed circuits.

In some implementations, the plurality of sensing electrodes can be electrically isolated from the ablation electrode.

In certain implementations, in the expanded state, the distal section of the balloon can be substantially hemispherical in the absence of a force on an external surface of the balloon. Additionally, or alternatively, in the expanded state, the proximal section of the balloon can be substantially frustoconical.

In some implementations, the combination of the ablation electrode and the plurality of sensing electrodes can span greater than about 90 percent of the entire surface area of the distal section of the balloon.

In certain implementations, the distal section of the balloon in the expanded state can define a maximum radial dimension of the balloon in the absence of an external force on a surface of the balloon.

Real time images of the balloon in the patient can be taken by, for example, such as x-rays and CT scanning. The balloon can be formed of a material that is opaque or translucent to the imaging system so that the balloon clearly appears in the images. The images of the balloon can be used to guide the surgeon while maneuvering the balloon into position against the region of the heart tissue to be ablated.

A liquid passage for saline solution can be included in the catheter such that saline continuously flows through the catheter, through the balloon, and from the outer surface of the balloon and into the vasculature including the heart. Apertures (e.g., irrigation openings) for the saline can be distributed throughout the outer surface of the balloon. The flow of saline solution continues throughout the period that the catheter is in the vasculature and the flow rate can increase while ablation energy is applied to the conductive material along the outer surface of the balloon. Providing liquid through the balloon to the expandable structure of the balloon can be useful for a variety of reasons, including to provide lubrication and irrigation between the outer surface of the balloon and tissue, cooling the conductive outer surface and heart tissue at the region of contact with heart tissue, and improving blood circulation over the surface of the balloon. In the area of contact between the conductive outer surface and heart tissue, the irrigation assists in circulating the blood from the contact area, cooling the contact area to ensure that the tissue in actual contact is not overheated, and providing more uniform heating of the heart tissue by cooling the center of the contact area that might otherwise overheat. Circulation of blood over the conductive outer surface assists in preventing overheating and stagnation of blood at the conductive outer surface. Blood can coagulate on the outer surface if it stagnates and overheats. The flow of saline solution can also reduce the risk of "steam-pop," the term used to describe the audible venting of gas that is a common complication during ablation therapy.

The ablation catheters described herein are advantageous compared to conventional ablation catheters, due in part to the balloon and large area ablation electrode disposed along the balloon. In conventional ablation catheters, the ablation electrode tends to be rigid and narrow due in part to the need to pass the electrode through an introducer sheath leading to patient vasculature. In other words, the size of a conventional electrode is limited by the size of the introducer sheath. Because the size of a lesion formed using an ablation electrode is limited by the approximate surface area of the ablation electrode that is brought into contact with targeted tissue, the narrow electrodes on a conventional ablation catheter typically form small area lesions. In addition, the small surface area of conventional ablation electrodes can concentrate current. As such, the electrical energy applied to targeted tissue via a narrow conventional electrode is often limited to avoid excessive current density in any region of the heart tissue. These limitations of conventional narrow tip ablation catheters can contribute to reduced size and depth of lesions.

In contrast with conventional narrow tip ablation electrodes, the balloons at the distal end of the ablation catheters described herein are collapsible and expandable. In its collapsed state, the balloon can slide through a sheath (e.g., an 8 Fr sheath, as is commonly used in cardiac procedures) that guides the balloon through the vasculature. The balloon can expand after extending beyond the sheath, such as by unfolding. The expansion allows the outer conductive surface area on the balloon to be substantially increased, e.g., an increase of 50 to over 100 percent. While expanded, a conductive outer layer on the balloon forms a large ablation electrode with a large area to contact heart tissue. The balloon can have a bulbous or semispherical shape which has a large surface area. More generally, at least a distal section of the balloon can have a surface area along a volume of revolution about a longitudinal axis defined by a catheter shaft coupled to a proximal section of the balloon. It should be appreciated that such a volume of revolution can form a balloon having a gently curved outer surface suitable for establishing a large contact area with the heart tissue. Because of the larger contact surface area, the lesions formed tend to be relatively large as compared to conventional ablation electrodes that have a smaller surface area.

In some implementations, the balloon can have a wall thickness of 0.20 mm or less. The conductive outer layer on the balloon can be a coated with a conductive material, such as a conductive ink. The conductive outer layer forms a single ablation electrode. The balloon thus has a single ablation electrode, and all of the ablation energy, e.g., RF energy, can be applied to the conductive outer layer.

To avoid having a disruption in the current path from the supply of current and through the outer conductive surface, there can be several electrical contacts, such as six, between the wiring providing RF energy and the outer conductive surface on the balloon. The electrical contacts can connect to wiring internal to the balloon and/or to the ablation catheter such that RF energy from the ablation energy generator can be delivered to the outer conductive surface. The electrical contacts can be symmetrically arranged with respect to the outer surface of the balloon. Several, such as three to six, electrical contacts can be arranged symmetrically in a forward hemisphere of the balloon. The electrical contacts can extend through openings in the wall of the balloon to connect the wiring to the conductive outer surface.

The balloon can be a hollow bulbous or spherical shape. As used here, a bulbous shape shall be understood to include any one or more of various different shapes in which at least a distal section of the balloon is substantially hemispherical and/or another shape having a gently curved or curvilinear surface. Additionally, or alternatively, the bulbous shape of the balloon can include a substantially hemispherical distal section and a substantially frustoconical proximal section. Such asymmetry of shape between the distal section and the proximal section of the balloon can be useful, for example, for achieving preferential deformation of the distal section of the balloon.

The material for the balloon can be a medical grade elastomeric material. In some implementations of the present technology, the wall thickness of the balloon can be between 0.05 mm and 0.20 mm in thickness. The wall of the balloon can have a tapering thickness, e.g., fold lines, to promote collapsing of the balloon and to allow for deformation of the balloon only at the site of tissue contact. By altering the wall thickness and the shape of the balloon to allow for deformation in a region of the balloon in contact with tissue, the surface area contact between the balloon and heart tissue can be generally increased.

The surface area of the conductive outer surface that contacts the tissue during treatment using ablation catheters configured in accordance with the present technology is larger than the surface area of many conventional ablation conductive outer surfaces. The larger contact surface area increases the area of heart tissue that can be ablated with each application of RF energy or other ablation energy. The lesion area can be broader than the physical contact area between the conductive outer surface and the heart tissue. The heating of heart tissue by the application of ablation energy often extends beyond the area of contact. Moreover, ablation and lesion formation can occur with minimal or no physical contact between the conductive outer surface and heart tissue due, for example, to electrical conduction through saline or blood of ablation energy from the conductive outer surface to heart tissue. A wide conductive outer surface should promote the conduction of ablation energy through saline and blood to heart tissue even if there is minimal or no physical contact between the outer surface and heart tissue. Because of the increased area of contact between the conductive outer surface and the heart tissue and the tendency of lesions to be larger than the contact area, the lesions formed by the conductive outer surface on the balloon become very large as compared to a lesion created by a conventional cardiac ablation tip.

The large contact area of the conductive outer surface and the deformable balloon are expected to reduce the risk that the ablation catheter will puncture, perforate, or otherwise unintentionally damage the heart tissue. The unintentional damage referred to in this paragraph is not the desired formation of a lesion due to the application of ablation energy. Rather, the unintentional damage referred to is the risk sometimes associated with conventional narrow cardiac ablation tips of puncturing, perforating, or otherwise damaging heart tissue by pushing the tip against the heart tissue. Because the risk of perforation, puncturing, or unintentionally damaging heart tissue is reduced, a cardiac ablation catheter having a large area conductive surface and elastomeric balloon can be pushed against the heart tissue with greater force, lessening and/or eliminating a need to control the application of pushing force against the heart tissue than with a conventional narrow tipped cardiac ablation catheter. Applying a larger physical force to the balloon to maintain good contact with the heart tissue provides (i) a better and more reliable contact, (ii) greater friction between the conductive outer surface and heart tissue than is expected with conventional narrow tipped cardiac ablation catheters, and (iii) a smaller risk of movement between the outer conductive surface and the heart tissue during application of ablation energy.

As discussed above, the balloon with its outer conductive surface can have a bulbous, spherical, or other generally curved outer shape. The generally curved outer shape promotes good contact between the outer conductive surface and heart tissue regardless of the angle between the balloon and the heart tissue in contact with the outer conductive surface. An elastically deformable balloon can also assist in providing good contact between the conductive outer surface and heart tissue even if the angle of contact is shallow or otherwise outside of a preferred range of angles of contact.

In contrast, the angle of contact between heart tissue and a conventional narrow tip ablation catheter is often important. If the angle is too shallow, the conventional narrow tip ablation catheter may not make good contact and can glance off the tissue during application of ablation energy. Glancing contact by a conventional narrow tip cardiac ablation catheter can result in poor, asymmetric contact with heart tissue that does not form suitable lesions. Similarly, movement of heart tissue due to the beating heart and respiration of the patient can cause a break or movement in the contact area between a conventional narrow tip ablation catheter and heart tissue during application of ablation energy.

With a conventional 3.5 mm ablation electrode tip, the maximum size of lesions formed by standard, short-duration applications of ablation are limited by the size of the tip. To achieve wider lesion formation with the conventional tip, power must be applied for an extended time of at least 30 to 60 seconds. Tissue needs to reach temperatures of at least 55 degrees Celsius (°C) to form the desired irreversible scarring or lesions. As tissue is heated, radiating and conductive heating carries the temperature increase deeper into tissue. Thus, increasing the time of ablation energy application to 30 to 60 seconds results in heated tissue conducting energy in every direction and warming all surrounding tissue and blood.

To achieve shallow and wide lesions, RF energy emitted from the conductive outer surface of a balloon configured in accordance with the present technology can be applied at high power and for short durations. High power and short duration applications maximize the potential of resistive heating for heating tissue while diminishing the effect of radiative and convective heating. In at least this application, 400 W (watts) and roughly 2.0 A (amps) can be delivered for short periods of approximately 10 seconds (e.g., 5 to 15 seconds, 8 to 12 seconds, etc.). Higher power delivery is contemplated for correspondingly shorter periods (e.g., 800 W and roughly 2.8 A for approximately 5 seconds). Short periods promote resistive heating of heart tissue and tend to be too short for extensive conductive heating of heart tissue. Resistive heating, also called ohmic heating, is a function of local current density and tissue resistivity, and occurs quickly within the tissue through which current is driven. The time constant that further governs lesion formation is dependent on tissue characteristics, such as heat capacitance. By relying more on resistive heating and less on conductive heating, the depth of lesion formation can be more easily calculated and controlled.

In thin heart tissue, such as tissue in the atria, high power and short duration application of ablation energy via the large area conductive outer surface can be useful to form transmural lesions without adversely affecting tissue around or beneath the ablated tissue, and mitigating risk of unintentionally damaging heart tissue or forming blood clots. Lesion depths of up to 5 mm can also be achieved, using short periods of energy application.

Short periods of ablation energy applications also assist in maintaining the contact location during ablation, and reduce the likelihood of the formation of blood coagulum, e.g., clots. Lesions formed by the large area conductive outer surface can have sizes of up to 15mm (millimeters) in diameter. Both shortening ablation energy applications periods and decreasing the total number of lesions needed to cover comparable surface areas (as a result of the large area of each lesion) can provide a practical advantage of reducing overall procedure time.

The large area of the conductive outer surface allows for high-power ablation applications. The large area allows for high power ablation without excessive current density at any specific point in the contact area with the heart tissue. The correlation between current density and local superficial heating places an upper bound on the power that can be safely applied per area of heart tissue. By increasing the area of contact with the heart tissue, the current density can be held within the upper bound while relatively high power is applied to the conductive outer surface for ablation. The saline irrigation can also cool a contact area of the heart tissue, which can serve to offset excessive local heating at the center of the contact area.

In some implementations, the conductive outer surface can deliver about six times the power of a conventional tip, while keeping the current density at the interface of the ablation electrode and the blood and tissue at about 65 percent of the upper bound of current density. Therefore, the larger conductive surface is expected to allow for greater power delivery and a more effective procedure due to lower heating at the ablation electrode interface. While energy inputs and parameters can vary from patient to patient based on physician preference, a larger conductive surface area allows for a wider range of parameters considered effective for use in comparison with the range of parameters considered effective for a conventional narrow tip cardiac ablation catheter.

The catheter ablation systems configured in accordance with the present technology can additionally comprise detection elements and sensing electrodes to assist a practitioner during insertion of the catheter through the vascular system, during positioning of the balloon against heart tissue, and/or during monitoring of the formation of lesions in the heart tissue. The detectors and sensors can provide feedback to the practitioner to ensure that energy application is progressing as desired and lesions are formed as intended. Signals received by detectors and sensors can be used to determine the location of the balloon, to determine whether the conductive outer surface is in contact with heart tissue, and/or to determine the area of contact between the outer surface and heart tissue. Additionally or alternatively, signals received by detectors and sensors can be used to monitor the application of ablation energy, to monitor the progress of lesion formation, and/or to monitor biological signals such as currents generated by the heart. The signals can be analyzed by computer systems, and the results of the analyses can be displayed on a digital display to the practitioner. The displayed information can include images showing the position of the balloon within the heart, an anatomical tissue target for lesion placement, and images of pulmonary veins and other organs to avoid when forming lesions. An electro-anatomical map can be generated and displayed showing the balloon and application of lesions in the heart tissue. Mapping or pretreatment of a target or a contact area prior to application of ablative energy can be used and/or applied to predict and/or improve the chances of success of the ablation therapy.

In some implementations, temperature and other factors at or near the surface of the tissue in contact with the conductive outer surface can directly indicate whether ablation has occurred. Conventional ablation catheters often employ one or more temperature sensors thermally coupled to ablation tips to read tissue temperature at these points. These readings, however, can be inaccurate as, instead of reflecting tissue temperature, they can reflect or be affected by the temperature of the ablation electrode or irrigation fluid used to cool the tissue at these points. In contrast with conventional ablation catheters, temperature sensors, such as thermistors, mounted to a balloon (e.g., along the distal section of the balloon) configured in accordance with some implementations of the present technology can be thermally insulated from irrigation fluid flowing through the structure and from the ablation electrode such that the temperature readings from the temperature sensors are accurate and reflect tissue temperature.

Temperature sensors distributed on the balloon in some implementations can be used to determine the region and area of the conductive outer surface in contact with heart tissue. For example, blood tends to cool the conductive outer surface more so than heart tissue. Thus, temperature sensors adjacent heart tissue detect a higher temperature relative to the other temperature sensors that are adjacent flowing blood. The temperature sensors with higher temperature readings, therefore, can correspond to contact with the heart tissue and can be used to map the area of contact between the conductive outer surface and heart tissue. The size of the lesion can be estimated by the number and position of the temperature sensors that provide higher temperature readings. An empirically determined algorithm can be generated for a specific balloon that estimates lesion size based, at least in part, on the number of temperature sensors reading a high temperature.

Ablation can be performed using temperature to control delivered power. The operator can set a maximum temperature (e.g., 65°C) and power titrated to achieve and maintain this temperature or a lower temperature, such as a temperature of 55°C. The temperature sensors can be used to estimate the temperature of the tissue. An empirically derived algorithm for each design of a balloon can be used to convert the temperature readings to tissue temperature. In the case of multiple temperature sensors (e.g., six) distributed across the balloon, the maximal temperature can be used for power titration. Furthermore, when tissue contact is detected at the location of each of the temperature sensors, only temperature from sensors known to be in tissue contact can be accounted for. Any of a number of other functions (e.g., average, median temperature) can be used to control the power and duration of the ablation energy.

Temperature can also be used to provide information on lesion progress. The temperature and number of sensors affected, as well as power and duration, can be used for this purpose. A range and distribution of more than a single temperature reading across the ablative tip can provide additional details or lesion characterizations. For example, for a given power and duration, a greater number of sensors showing a rise in temperature can result in a lesion assessment suggesting greater width and depth. It can be inferred that a lesion forming where only one sensor detects a temperature rise is smaller than one in which multiple sensors detect a temperature rise.

Tissue impedance at a localized region of tissue can provide powerful insight into the state of the tissue targeted for ablation. Sensing electrodes, such as pairs of concentric electrodes, are located on the outer surface of the balloon. The sensing electrodes provide localized sensing or measurements of regions of the conductive outer surface and of regions of heart tissue near the sensing electrodes. A sensing electrode with a pair of concentric electrodes provides localized sensing by, for example, measuring a voltage difference between the pair of concentric electrodes. The voltage difference is affected by just the small region between the pair of electrodes.

In certain implementations, the conductive outer surface includes one or more concentric electrodes that has a central conductive component surrounded by an annular conductive component. This arrangement can reduce or minimize noise and far field effects in bipolar signals constructed with these two electrodes, which can allow for improved electrograms. Such electrodes can also provide bipolar electrograms that are more consistent across electrode to tissue orientation than those produced with conventional bipolar electrodes. By (i) driving current between the two concentric electrodes (e.g., in a frequency range of 5 to 50 kHz (that is well below the frequency of the ablation energy)) and (ii) monitoring values resulting from the driven current ( e.g., impedance values, voltage values, and/or current values), this electrode arrangement can also allow for improved detection of contact between the conductive outer surface (e.g., a region of the conductive outer surface including the concentric electrode) and tissue.

At each position configured with a sensing electrode having a concentric pair of electrodes, detection of tissue contact is made possible by passively detecting impedance based on the current flow through the electrodes and the voltage between the electrodes. A change in the impedance at the sensing electrode can be used to indicate (i) tissue contact near the region of the outer conductive surface of the sensing electrode and (ii) formation of a lesion in the tissue near the sensing electrode. For example, a gradual change in the impedance in the path between the concentric electrodes of a sensing electrode can be used to detect lesion formation. A comparison of the impedance measured at one of the sensing electrodes with the impedance at another one of the sensing electrodes can be used to determine the area of contact between the conductive outer surface.

Impedance measured through tissue is inversely correlated to both tissue temperature and lesion formation. As such, a measurement of tissue impedance can reveal if tissue has been ablated and whether the ablation is relatively superficial or transmural. Transmural lesion formation is generally considered necessary to interrupt the electric signals that propagate arrhythmias. Monitoring impedance to detect the moment a lesion has become transmural can accurately determine when the application of ablation energy can be terminated. In some implementations, impedance is an indicator of lesion formation.

In some implementations, the thermistors can be imbedded behind the concentric electrode pair to increase insulation and to consolidate use of circuitry and space. Lesion size can be estimated as a function of known or detected values, such as a function of temperature measurements taken from temperature sensors distributed in the balloon. For example, lesion size as a function of multiple temperature measurements can be characterized as:

$$Lesion\ Size = f(T_1, T_2 \dots T_i, i, I, t)$$

where $T_1, T_2 \dots T_i$ are measured temperatures, $i$ is the ablation current, $t$ is lesion duration and $I$ is tip to return electrode impedance. The function, $f$, can be a logarithmic function. Accurate measurements of tissue impedance or temperature are made possible by the concentric electrodes (that provide localized sensing), by the internal electrodes (that provide signals compared to the outer concentric electrodes), and by the temperature sensors (that are isolated from the irrigation fluid and configured to reliably sense temperature indicative of tissue temperature).

Other ablation catheter systems described herein are expected to allow for improved detection and monitoring of contact between the ablation conductive outer surface and the tissue to be ablated. In certain implementations, for example, the ablation conductive outer surface is conformable to tissue and includes a radio-opaque material. As a result, deformation of the tip resulting from contact with the tissue to be treated can be easily visualized and detected under fluoroscopy.

In some implementations, the ablation conductive outer surface is equipped with sensing mechanisms that can be used to monitor the shape, or internal fluid volume of the conductive outer surface. This information can be used to monitor the degree and location of contact between the conductive outer surface and the tissue (sometimes referred to as tip to tissue coupling) and can thus be used to infer certain characteristics, such as size and depth, of the resulting lesion. This can provide the physician with greater control over catheter manipulation and can result in a more accurate and effective application of therapy.

Finally, in some implementations, a pressure sensor can be placed in communication with fluid at the conductive outer surface. This pressure sensor can advantageously be used to detect tissue contact when irrigation holes are occluded, and can be used to provide feedback to a pump providing a predetermined or appropriate level of irrigative flow.

For clarity in this application, the balloon can be referred to in some implementations as the deformable or conformable tip. In addition, it is to be appreciated that the ability of the tip to deform allows the surface of the tip to conform to tissue shapes and contours. Lastly, the term conformal tip, describes a tip with similar features, which conforms to corresponding points of tissue, as a result of tissue contact and shape change.

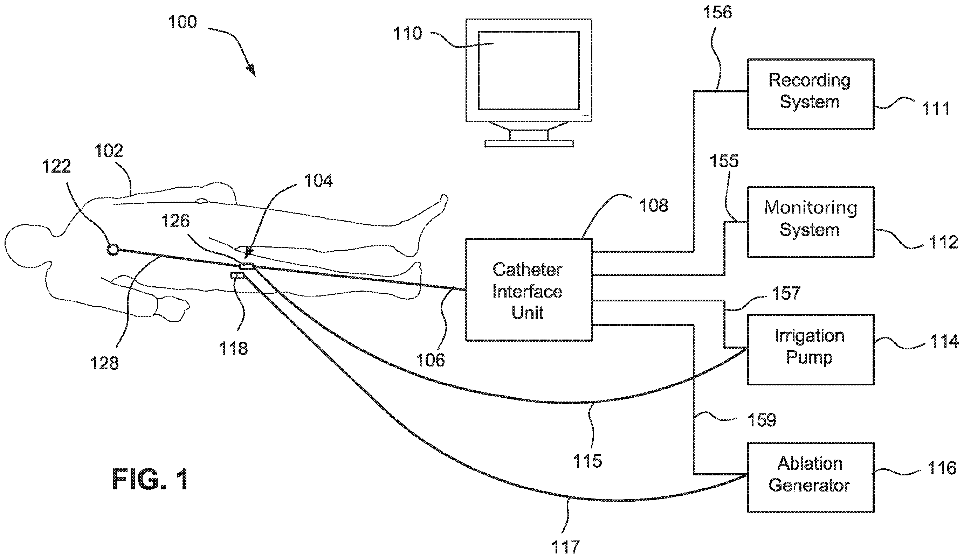

Certain details are set forth in the following description and in Figures 1-17 to provide a thorough understandings of various implementations of the disclosure. Other details describing well-known structures and systems often associated with ablation catheters and associated systems and methods, however, are not set forth below to avoid unnecessarily obscuring the description of various implementations of the disclosure.

Many of the details, dimensions, angles, and other features shown in Figures 1-17 are merely illustrative of particular implementations of the disclosure. In addition, those of ordinary skill in the art will appreciate that further implementations of the disclosure can be practiced without several of the details described below.

B. Selected Implementations of Ablation Catheters and Related Systems and Methods, whereby the systems and methods are not covered by the claimed invention

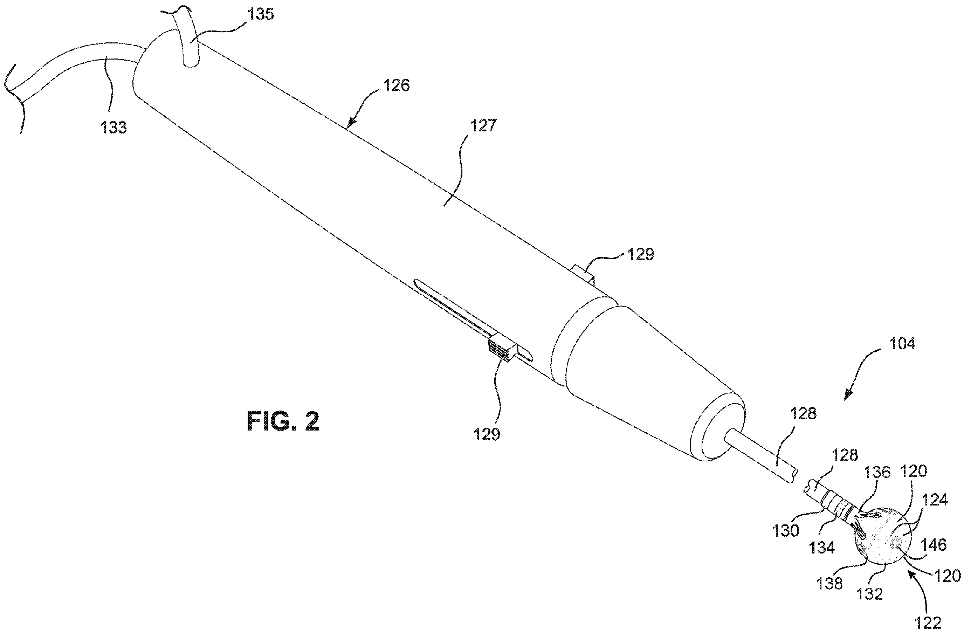

Figure 1 illustrates an ablation system 100 configured in accordance with an implementation of the present technology being utilized for cardiac ablation treatment of a patient 102. The ablation system 100 includes an ablation catheter 104 having a handle 126, a catheter shaft 128, and an expandable element or balloon 122. The ablation catheter 104 is connected via an extension cable 106 to a catheter interface unit 108 having a display 110. A monitoring system 112, recording system 111, an irrigation pump 114, and an ablation generator 116 are connected to the catheter interface unit 108 via electrical cables 155, 156, 157, and 159, respectively. The irrigation pump 114 is also removably and fluidly connected to the ablation catheter 104 via fluid line 115. The portion of the fluid line 115 to be inserted into the patient is dimensioned to fit within the ablation catheter 104 (e.g., in fluid communication with an irrigation lumen defined by the catheter shaft 128) and is fluidly connected to the balloon 122, illustrated in greater detail in Figures 2-4. The ablation generator 116 can also be connected via a wire or cable 117 to a return electrode 118 attached to the skin of the patient 102. The recording system 111 can be used in support of the ablation treatment. The monitoring system 112 can be used prior to or during an ablation treatment in order to monitor the cardiac tissue of the patient 102, and determine which region or regions of the cardiac tissue require ablation.

During an ablation treatment, the ablation generator 116 controls and provides energy (e.g., electrical energy in the RF range, at approximately 350-600 kHz) to an ablation electrode (not shown). As described in greater detail below, the ablation electrode is a continuous conductive layer applied to and disposed along the outer surface of the balloon 122. At the same time energy is delivered to the ablation electrode, the irrigation pump 114 pushes irrigation fluid (e.g., saline) into the balloon 122 of the ablation catheter 104. For example, the balloon 122 can, in some implementations, define a volume in fluid communication with the irrigation lumen defined by the catheter shaft 128. The irrigation fluid is released from the balloon 122 via multiple irrigation orifices (not shown) and serves (i) to cool the ablation electrode and/or the outer surface of the balloon 122 (especially a portion of the ablation electrode and/or a portion of the outer surface of the balloon 122 in contact with tissue), and (ii) to move blood away from the ablation electrode and/or the outer conductive surface. Additionally, or alternatively, the volume defined by the balloon 122 can be in thermal communication with the ablation electrode disposed along the balloon 122 through, for example, the material of the balloon 122 such that fluid in the volume of the balloon 122 cools the ablation electrode The energy provided by the ablation generator 116 to the ablation electrode heats and ablates region(s) of heart tissue into which the energy is directed.

Referring to Figure 2, the handle 126 of the ablation catheter 104 is attached to a proximal end portion of the catheter shaft 128, and the balloon or expandable element 122 is attached to a distal end portion of the catheter shaft 128. The catheter shaft 128 is distinguished from a housing 127 of the handle 126, as it is dimensioned for entry into the patient body. The catheter shaft 128 can be formed of any of various different biocompatible materials that provide the catheter shaft 128 with sufficient sturdiness, but also flexibility to allow the catheter shaft 128 to be navigated through blood vessels of the patient 102 (Figure 1). Examples of suitable materials from which the catheter shaft 128 can be formed include polyether block amide sold under the trademark PEBAX and commercially available from Arkema, Inc. of King of Prussia, PA, nylon, polyurethane, thermoplastic polyurethanes sold under the trademark PELLETHANE and commercially available from Lubrizol Corp. of Wickliffe, OH, and/or silicone. In certain implementations, the catheter shaft 128 includes multiple different materials along its length. The materials can, for example, be selected to provide the catheter shaft 128 with increased flexibility at the distal end, when compared to the proximal end of the catheter shaft 128. The catheter shaft 128 can also include a tubular braided element that provides torsional stiffness while maintaining bending flexibility to one or more regions of the catheter shaft 128. Furthermore, the shaft material can contain radio-opacifying agents such as Barium Sulfate or Bismuth, to allow fluoroscopic visualization.

The handle 126, including the housing 127 and a lever mechanism 129, remains external to the patient's body. The lever mechanism 129 can be operated to deflect or steer the distal end portion of the catheter shaft 128. The proximal end portion of the handle 126 includes a fluid line connector 133 (e.g., a luer connector) to which the fluid line 115 (Figure 1) extending from the irrigation pump 114 (Figure 1) can be connected to deliver saline to the ablation catheter 104. The proximal end portion of the handle 126 also includes an electrical connector 135 to which the extension cable 106 (Figure 1) extending from the catheter interface unit 108 (Figure 1) can be connected. This connection allows (i) electrical energy to be delivered from the ablation generator 116 (Figure 1) to the ablation catheter 104 and (ii) electrical and temperature data to be collected and captured from the balloon 122 via the catheter interface unit 108.

The handle 126 can be attached to the proximal end portion of the catheter shaft 128 using various attachment techniques, such as adhesive bonds, thermal bonds, and mechanical connections. The catheter shaft 128 includes a tube (not shown) that is in fluid communication with the fluid line 115 extending from the irrigation pump 114 and serves to carry saline from the proximal end of the ablation catheter 104 to the balloon 122. The catheter shaft 128 also includes electrical wires connected to various electrodes and sensors on the balloon 122. The electrical wires can be bundled or wrapped to increase their durability under flexure or bending. The catheter shaft 128 further includes wires (i) that are attached at their distal ends to a ring (not shown) secured to the distal end portion of the catheter shaft 128 and (ii) that are attached at their proximal ends to the lever mechanism 129 of the handle 126 to allow the user or practitioner to apply tension to the wires to deflect the distal end portion of the catheter 104 for steering purposes.

In some cases, a magnetic position sensor (not shown) can also be positioned at the distal end portion of the catheter shaft 128. The position sensor can, for example, include one or more coils configured to detect signals emanating from magnetic field generators. One or more coils for determining position with five or six degrees of freedom can be used. For six degrees of freedom, two coils that are parallel to each other can be used at the tip of the catheter shaft 128. Alternatively, two coils that cross each other can also be used. The magnetic field detected by the position sensor can be used to determine the position of the distal end of the catheter shaft 128.

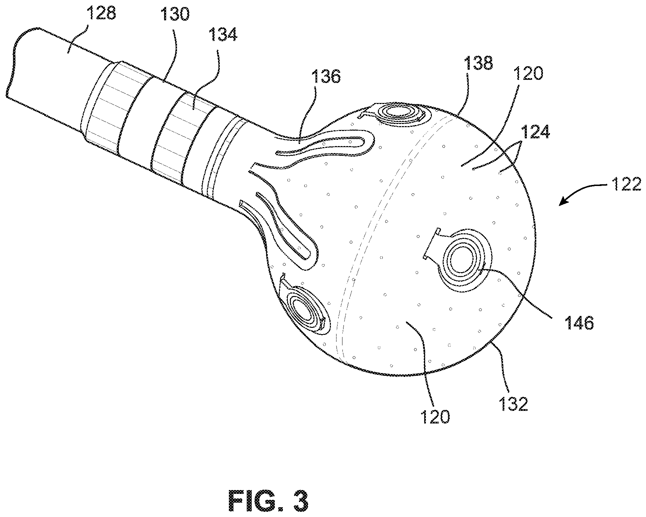

As illustrated in Figures 2 and 3, the catheter shaft 128 is fluidly connected to the balloon or expandable element 122. The balloon 122 includes a proximal section and a distal section. At least the distal section of the balloon 122 can be semi-compliant to at least partially deform upon contact with tissue. In certain implementations, the distal section of the balloon 122 can be more compliant than the proximal section of the balloon 122 in one or more directions (e.g., in response to an axial force on the balloon 122). For example, the distal section of the balloon 122 can preferentially deform upon contact with tissue while the proximal section of the balloon 122 provides structural rigidity to facilitate controlling placement of the distal section of the balloon 122. In general, it should be appreciated that differences in compliance between the distal section of the balloon 122 and the proximal section of the balloon 122 can be achieved through any of various different modifications to the balloon 122 and, thus, can be achieved through the use of different materials, different material thickness (e.g., with the thickness of the proximal section of the balloon 122 being greater than a thickness of the distal section of the balloon 122), support features, and/or combinations thereof.

The balloon 122 includes a neck 130 disposed along the proximal section and a body 132 disposed along at least the distal section of the balloon 122 and, in certain instances, extending from the proximal section to the distal section of the balloon 122. The neck 130 of the balloon 122, is connected to the distal end portion of the catheter shaft 128. In some cases, an electrically conductive adhesive can be used to electrically connect the electrical wires inside the catheter shaft 128 to further points in or on the balloon 122. An electrically conductive adhesive can also be used between the neck 130 and the catheter shaft 128 to secure those components together while enabling energy to freely pass between those components. An electrical connection can also be formed at the outer surface of the neck 130 where the balloon contacts the catheter shaft 128. In one alternative implementation, as the body 132 of the balloon 122 comes into side-contact with tissue, a lateral force at the contact region will result in a bending moment at the neck 130. In such implementations, it can be advantageous to avoid forming electrical connections at the neck 130, as increased strain or design deflection of the neck 130 can result in an increased number of discontinuities.

Not shown are alternative means for attaching and fixing the balloon 122 to the catheter shaft 128. For example, in at least one implementation the catheter shaft 128 and/or the balloon 122 can be swaged to connect the catheter shaft 128 to the neck 130 of the balloon 122. Other connection methods can also comprise threaded fittings, conical fittings, tapered connections, over-molding elastomers, use of coupling configurations, and/or combinations thereof. Additionally or alternatively, an appropriate (e.g., RTV silicone) adhesive can be used to bond the neck 130 to the distal end portion of the catheter shaft 128. When used, an electrically conductive adhesive, in addition to providing a securing function, can facilitate transmission of electrical energy from a conductive ring within the distal end portion of the catheter shaft 128 to the ablation electrode 120 via the inner surface of the neck 130. Examples of electrically conductive adhesives that can be used include silver-filled silicone RTV adhesives (e.g., Loctite 3888).

The body 132 of the balloon 122 can be expanded to a size larger than conventional conductive outer surfaces, while also being deliverable to a region of tissue to be treated. For example, one or both of the body 132 and the neck 130 of the balloon 122 can be self-expandable such that the balloon 122 is expandable from a collapsed state to an expanded state in the absence of a force on an external surface of the balloon 122. Additionally, or alternatively, at least a portion of the balloon 122 can be inflatable (e.g., under the force of irrigation fluid moving through the balloon 122) from a collapsed state to an expanded state. In the expanded state of the balloon 122 (whether expanded through self-expansion, inflation, or both), the ablation electrode 120 can extend along at least a distalmost point of the balloon 122 in the absence of a force on an external surface of the balloon 122. Similarly, in the expanded state of the balloon 122, whether expanded through self-expansion, inflation, or both, the ablation electrode 120 can extend along at least a radial-most point of the balloon 122 in the absence of a force on an external surface of the balloon 122. Such placement of the ablation electrode 120 along one or both of the distalmost point and the radial-most point of the balloon 122 can, for example, facilitate delivery of energy along regions of the balloon 122 most likely to make initial contact with tissue and, thus, can facilitate accurate placement of the ablation electrode 120 at a treatment site.

In certain implementations, the body 132 of the balloon 122 has a diameter of 2.0 mm to 25 mm (e.g., 5 mm, 10 mm, and 12 mm). The wall thickness of the body 132 can have a thickness of 10 $\mu$m to 1000 $\mu$m (e.g., 25 $\mu$m to 800 $\mu$m, 25 $\mu$m to 400 $\mu$m, 50 $\mu$m- 500 $\mu$m, 100 $\mu$m - 1000 $\mu$m, and so forth). The body 132 of the balloon 122 can be formed of one or more biocompatible materials that allow the body 132 to conform to tissue and to be collapsed within a sheath, while also sufficiently durable in its expanded form. In some implementations, the material from which the body 132 and the neck 130 are formed has a durometer of 10 shore A to 70 shore D (e.g., 40 shore A). In certain implementations, the body 132 and the neck 130 can comprise silicone, fluorosilicone, urethane, polyethylene, thermoplastic polyurethane, polyvinyl chloride, polyethylene terephthalate, PEBAX, nylon, polyurethane, and/or combinations thereof. In some implementations, the material from which the body 132 is formed can be loaded with a radiopaque additive, such as a barium sulfate or bismuth. The radiopaque additive permits the body 132 to be visualized using fluoroscopy during an ablation treatment. In certain implementations, for example, the body 132 has a diameter of 8 mm, a wall thickness of 150$\mu$m, and is formed of silicone loaded with barium sulfate. In other implementations, however, the body 132 may have a different size/arrangement.

As shown in Figures 2 and 3, a continuous conductive layer is applied to the outer surface of the balloon 122. This layer, electrically connected to the ablation generator 116 (Figure 1), forms the ablation electrode 120 of the ablation catheter 104. The ablation electrode 120 can be formed, for example, of conductive ink that coats at least a portion of the outer surface of the body 132 of the balloon 122. In some implementations, a portion or the entire surface of the neck 130 of the balloon 122 can also be covered with the conductive layer. The ablation electrode 120 is flexible and is configured to expand and collapse with the balloon 122 without affecting the ability of the ablation electrode 120 to transmit energy. The ablation electrode 120 can include one or more conductive materials, such as silver, carbon, gold, silver-coated glass beads, graphene. In other implementations, the ablation electrode can be formed of conductive ink comprising silver flakes that is sputtered onto the balloon 122.

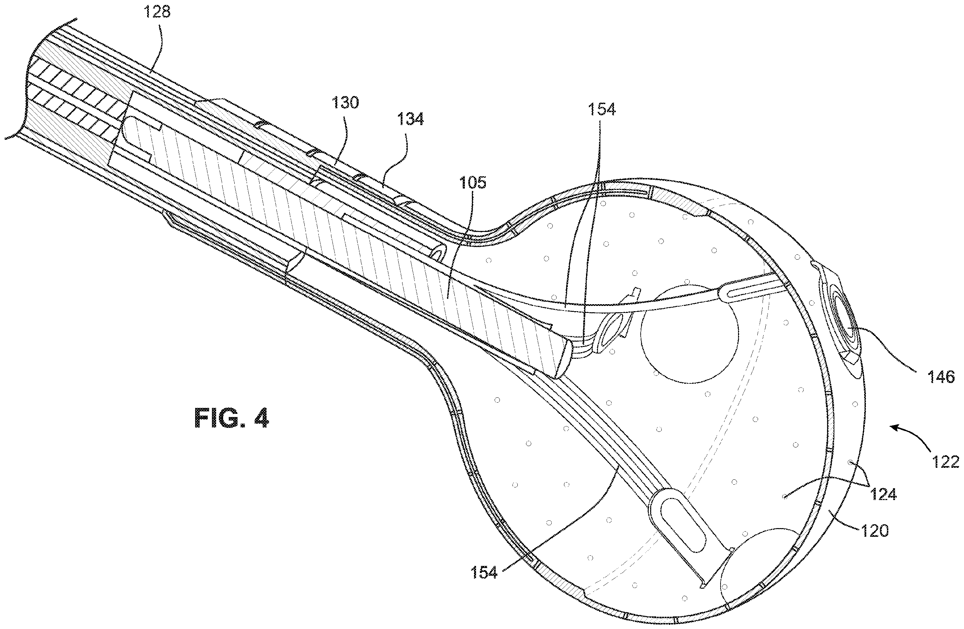

As shown in Figure 4, a printed circuit 154 can include one or more (e.g., six) conductive strips that can be formed of Nitinol and can be used to deliver ablative energy to the outer conductive surface (e.g., the conductive ink) that forms the ablation electrode 120. In this case, a wire (not shown) running down the catheter shaft 128 is connected to the printed circuit 154 inside the balloon 122. Each conductive strip of the circuit 154 can provide some added rigidity to the elastomeric balloon 122. At the end of each of the conductive strips, the elastomer insulating material of the balloon 122 can be locally thinned or removed. Direct electrical connections to the surface of the balloon 122 can be made through the balloon 122 to sensing elements 146 and/or to the ablation electrode 120.

In a specific implementation, elastomer can be overmolded onto electrical connections formed between the printed circuit 154, the ablation electrode 120, and/or the sensing elements 146. The conductive ink can be applied such that it contacts the inner conduit directly at each location an electrical contact is formed. As a result, ablation current is introduced to the surface of the balloon 122 through each point of electrical connection. One benefit of this approach is that it provides redundancy should an electrical connection to the conductive ink fail. It also provides a convenient means to transition from a signal residing inside the catheter shaft 128 to the ablation electrode 120 at the outer surface of the balloon 122.

Referring to Figures 2-4, the balloon 122 includes a neck 130 mounted to the catheter shaft 128. A neck-mounted electrode 134 can be arranged as a ring around the catheter shaft 128 or configured as needed. The neck-mounted electrode 134 can also be separately or concurrently used to support an impedance or current based localization system. A location signal, either passively measured or driven between another sensing electrode 146 and the neck-mounted electrode 134, is used to determine deflection of the neck 130 of the balloon 122. Deflection of the neck 130 indicates contact between the ablation electrode 120 and heart tissue.

In addition, the sensing electrodes 146 can be used to relate location signals (e.g., potential field measurement or impedance) measured through them. In an alternative implementation, the location could be determined using an independent localization system (e.g., a magnetic localization system described above) to support a hybrid magnetic and impedance based localization.

Referring to Figures 2-5, a plurality of sensing electrodes 146 can be arranged on the surface of the balloon 122 and electrically insulated from the ablation electrode 120. For example, the plurality of sensing electrodes 146 can include six electrodes 146. Three of the sensing electrodes 146 can be positioned equidistant from each other on the proximal section of the balloon 122, and three of the sensing electrodes 146 can be positioned along the distal section of the balloon 122, symmetrical across the imaginary equator of the balloon 122.

In general, each sensing electrode 146 is bounded by the ablation electrode 120. For example, the ablation electrode 120 can extend between each sensing electrode 146 and each of the other sensing electrodes 146. With the ablation electrode 120 bounding each of the sensing electrodes 146, the conditions sensed by the plurality of sensing electrodes 146 can accurately represent local conditions along the ablation electrode 120 as energy is delivered from the ablation electrode 120 to tissue.

In certain implementations, the plurality of sensing electrodes 146 can have a radial thickness substantially similar to a radial thickness of the ablation electrode 120. Such uniformity of radial thickness can be useful, for example, for forming a substantially uniform surface along the balloon 122. A substantially uniform surface can facilitate placing the plurality of sensing electrodes 146 proximate to a position or positions of contact between the ablation electrode 120 and tissue.

The ablation electrode 120 and the plurality of sensing electrodes 146 can span substantially the entire surface (e.g., greater than about 90 percent of the surface area) of the distal section of the balloon 122, allowing for (i) electrically insulating material between the plurality of sensing electrodes 146 and the ablation electrode 120 and/or (ii) irrigation orifices 124. That is, substantially the entire surface area of the distal section of the balloon 122 can be used for delivering energy or monitoring the energy being delivered. Further, or instead, independent of an orientation of the distal section of the balloon 122 to tissue, any line of contact between the distal section of the balloon 122 and the tissue can be substantially continuously spanned by the ablation electrode 120, one or more of the sensing electrodes 146, or a combination thereof. Thus, it should be appreciated that, relative to an ablation electrode requiring a specific orientation, substantially spanning the entire surface of the distal section of the balloon 122 with the ablation electrode 120 and the plurality of sensing electrodes 146 can offer certain advantages with respect to ease of positioning the balloon 122 relative to tissue to be treated.

In certain instances, the distal section of the balloon 122 in an expanded state can define a maximum radial dimension of the balloon 122 in the absence of an external force on a surface of the balloon 122. Thus, for example, the maximum radial dimension of the balloon 122 can be substantially spanned by the ablation electrode 120, the plurality of sensing electrodes 146, and a combination thereof.

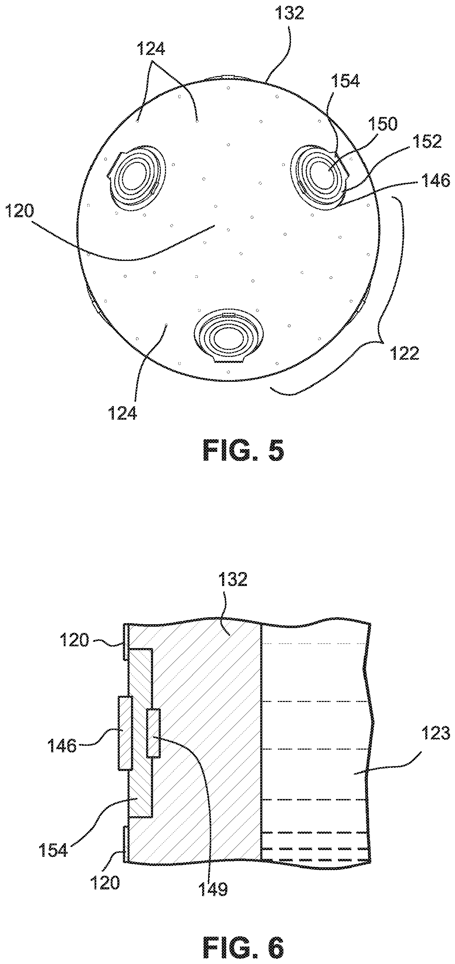

Referring to Figure 5, each of the sensing electrodes 146 includes a central conductive component 150 and an annular conductive component 152 that surrounds the central conductive component 150. The conductive components 150, 152 are concentric conductive electrodes and are attached to the flexible printed circuit 154, which is attached to the body 132 of the balloon 122. Each of the conductive components 150, 152 is connected to a wire (not shown) that extends through the catheter shaft 128 (Figures 1-4) to the catheter interface unit 108 (Figure 1) for transferring data signals from the conductive components 150, 152 to a control unit (e.g., processor such as a microprocessor or other logic processor) in the catheter interface unit 108. Alternatively, a multiplexing integrated circuit (not shown) within the balloon 122 can allow for the use of a single wire (not shown) through the catheter shaft 128.

The conductive components 150, 152 are typically thin pieces of conductive materials. Examples of suitable materials from which the conductive components 150, 152 can be formed include silver, copper, gold, nickel, aluminum, and/or combinations thereof. In certain implementations, the conductive components 150, 152 can be coated with a specialized coating, such as sputtered or electroplated iridium oxide. This coating can reduce the electrical impedance of the saline-electrode interface, thereby reducing noise.

The central conductive component 150 typically has the same surface area as the annular conductive component 152. This can permit the conductive components 150, 152 to have nearly identical impedance to the surrounding blood and can (i) limit noise picked-up when the conductive components 150, 152 are used to provide bipolar measurements and (ii) reduce emitted noise when the conductive components 150, 152 are driven with a current. In some implementations, each of the conductive components has a surface area of 0.25 mm$^2$ to 1 mm$^2$ (e.g., 0.5 mm$^2$).

The concentric design of the conductive components 150, 152 reduces and, in some cases, eliminates the effect of the electrode pair orientation relative to tissue on a collected signal when the electrode 146 is pressed against the tissue. Because the expandable structure of the balloon conforms to a tissue surface, conductive components 150, 152 can be pressed flat against a tissue surface, which further reduces orientation impact on a bipolar signal constructed with the conductive components 150, 152. In addition, the far field effect is minimized because the relatively small size and close spacing between the conductive components 150, 152 helps to ensure that they measure signals from the underlying tissue. The deformable material of the balloon 122 also minimizes the far field effect of signals picked up from neighboring tissue. Furthermore, the separation of these conductive components 150, 152 from the ablation electrode 120 can improve the accuracy of detection during ablation, as compared to conventional devices that use the same electrode to measure signal and to deliver therapy.

In combination, these properties are expected to provide localized electrograms that are consistent and easily interpreted. Conventional bipolar electrode pairs often have mismatched surface areas (e.g., where the surface area of the ablation electrode exceeds the surface area of the other electrodes) leading to added noise, a bipolar signal with an amplitude and sign that are dependent on tip-tissue orientation, larger electrode sizing that results in more far-field signals from adjacent tissue, and more noise as ablative energy is delivered to one electrode in the pair.

When desired, the body 132 of the balloon 122 can be formed of a material chosen to affect certain characteristics. For example, the body 132 can be formed of specific polymers, formed from combinations of polymers, or loaded with a material to alter thermal or electrical conductivity. A body 132 with a relatively high thermal conductivity and a relatively low conductivity can improve the electrical insulation of sensing electrodes 146. Improving thermal conductivity to the polymer body 132 can alter the thermal conductivity of the body 132, which can help to distribute heat throughout the material of the balloon 122 and prevent localized hot spots. Conversely, thermal conductivity can be made relatively poor to further insulate sensors and/or electrodes imbedded within the body 132.

In one implementation, as shown in Figure 6, thermistors 149 can be positioned at inner surfaces of the flexible printed circuit 154, opposite the central conductive components 150, 152 (Figure 5) of the sensing electrodes 146. The thermistors 149 can be thermally isolated and can be used to detect the temperature of their associated sensing electrodes 146. Any of various types of thermistors, resistance thermometers, and thermocouples can be used. In certain implementations, the thermistors 149 are discrete negative temperature coefficient elements. Because the thermistors 149 are separated from the sensing electrodes 146 by only the thin material (e.g., polyimide) of the flexible printed circuit 154 and are separated from the ablation source and irrigation fluid (e.g., saline 123) by the thicker material (e.g., silicone) of the balloon 122 with a relatively low thermal conductance, the electrical resistance of the thermistors 149 can be used to accurately estimate the temperatures of the sensing electrodes 146. Since the sensing electrodes 146 are exposed along the outer surface of the balloon 122 and can be placed in direct contact with tissue of the patient during use, their temperature can provide an accurate indication of the tissue temperature during treatment. Tissue temperature has been found to be a good indicator of ablation lesion formation. In some implementations, temperature data from the thermistors can also be used as a safety control to stop ablation delivery if overheating (e.g., >80°C) occurs.

The flexible printed circuits 154, to which the sensing electrodes 146, the thermistors 149, and/or optional inner electrodes (not shown) can be attached, can be formed on one or more biocompatible substrates, such as polyimide or parylene. Metallization layers can comprise conductive elements including silver, copper, gold, and/or combinations thereof. An outer layer can include materials such as polyimide, parylene, and/or others. In some examples, rather than a flexible printed circuit 154, the sensing electrodes 146 and thermistor 149 can be applied using selectively applied ink for traces and electrodes. The ink can be sprayed or sputtered on the distal end of the balloon 122 with masks selectively uncovering trace and electrode areas. circuits can be formed by additional methods and are configured to be small, flexible, and conductive. Alternatively, the ink can be applied using micropenning techniques. While certain ablation electrodes 120 have been described as including conductive ink, in some implementations other types of conductive layers can be used.

As shown in Figures 2-5, after forming the body 132 and the neck 130 of the balloon 122, the material of the body 132 can be processed in a desired pattern to define irrigation orifices 124. The irrigation orifices 124 can be formed using any of various suitable techniques, including laser drilling, and mechanical cutting (e.g., using a hole puncher or drill). In at least one implementation, the irrigation orifices 124 are patterned in a manner suitable to supply consistent irrigation to all areas of the balloon 122 (e.g., along each of the distal section of the balloon 122 and the proximal section of the balloon 122). In one specific example, a plurality of irrigation orifices 124 (e.g., 112 irrigation orifices), each with a diameter of $50\mu$m, has been found to provide appropriate irrigation. In other examples, however, a different number of irrigation orifices 124 may be used and/or the irrigation orifices 124 may have a different diameter.

As an additional or alternative example, at least some of the irrigation orifices 124 can extend through the ablation electrode 120. Such placement of the irrigation orifices 124 through the ablation electrode 120 can be useful, for example, for providing cooling at one or more points of contact between the ablation electrode 120 and tissue. Further, or instead, at least some of the irrigation orifices 124 can be positioned to direct irrigation fluid (e.g., saline 123, as shown in Figure 6) in a proximal direction relative to the balloon 122. Such positioning of at least some of the irrigation orifices 124 in the proximal direction can reduce the likelihood that contact between the balloon 122 and tissue will occlude all of the irrigation orifices 124. Thus, for example, in instances in which contact between the balloon 122 and tissue occludes irrigation orifices 124 along the distal section of the balloon 122, irrigation fluid can continue to flow through the balloon 122 and through the irrigation orifices 124 positioned to direct irrigation fluid in the proximal direction relative to the balloon 122.

Referring again to Figure 6, the sensing electrodes 146, the thermistors 149, and/or the optional inner electrodes (not shown) can be attached to the balloon 122 using a compliant adhesive, such as a RTV silicone. Alternatively, or additionally, the sensing electrodes 146, the thermistors 149, and/or the optional inner electrodes can be attached to the balloon 122 via mechanical retaining features (e.g., tabs) (not shown) added to the perimeter of the flexible circuit 154 that can be inserted into mating holes or slot features molded into the balloon 122. Alternatively, or additionally, the sensing electrodes 146, the thermistors 149, and/or the optional inner electrodes can be molded or overmolded into the balloon 122 during its manufacture. Alternatively, staples or sutures, e.g., of Nylon, can be used to fasten the sensing electrodes 146, the thermistors 149, and/or the optional inner electrodes to the balloon 122. In some implementations, an additional thermistor 149 can be mounted inside the balloon 122 in thermal communication with the irrigation fluid (e.g., with the saline 123).

In certain implementations, a pressure sensor (not shown), such as a MEMS piezo-resistive element, for measuring internal fluid pressure can also be provided in the balloon 122. The pressure sensor can be positioned on the deformable wall of the body 132 such that it is detecting the pressure difference between irrigation fluid (e.g., saline 123) and blood. Alternatively, the pressure sensor can be positioned internally within the balloon 122 to measure irrigation fluid pressure in the balloon. Pressure changes are less apparent when measured with a pressure sensor at the proximal end of an irrigation lumen 105 (Figure 4) defined by the catheter shaft 128 (Figure 1-4) due to the high fluid flow resistance (which increases in a long, narrow tube) in the irrigation lumen 105. As such, it can be advantageous to have a pressure sensor near distal end of the irrigation lumen 105. The internal fluid pressure sensor can be connected to the catheter interface unit 108 (Figure 1) via a wire or cable 106 to transmit pressure data to the control unit of the catheter interface unit 108. This pressure data can be used by the control unit to control the flow rate of irrigation fluid delivered to the balloon 122 by the irrigation pump 114 (Figure 1). In implementations with uniform hole patterns of irrigation orifices 124 (Figures 2-4), a pressure sensor would provide some indication as to the amount of surface area engaging with tissue. If irrigation orifices 124 are pressed against tissue, fluid resistance will increase and subsequently cause an increase in pressure, as well as balloon 122 firmness. The measurements provided by a pressure sensor can also be used to titrate irrigation. Finally, the irrigative flow can be adjusted by a user or practitioner. In other implementations, flow might change automatically based on power application or via sensor feedback.

Referring again to Figure 1, the catheter interface unit 108 acts as the interface between the ablation catheter 104 and other instrumentation of the ablation system 100. The monitoring system 112, the recording system 111, the irrigation pump 114, and the ablation generator 116 are connected to the catheter interface unit 108 via electrical cables 155, 156, 157, and 159, respectively. The catheter interface unit 108 is operably connected to the ablation generator 116 such that the catheter interface unit 108 can receive data from the various sensors or can control the output of or communicate with the ablation generator 116 according to a predetermined scheme or user input. The catheter interface unit 108 can include a processor (e.g., control circuit), electronic hardware, non-transitory memory storing software, firmware, and/or any other logic control adapted to perform the features discussed herein. In some implementations, the ablation generator 116 includes its own controller for governing the generator 116 based on data received from the sensors at the balloon 122.

The catheter interface unit 108 can also include a current source that can generate a signal at a frequency above the cardiac band (e.g., 5 kHz). The signal can be a sinusoidal or other waveform signal with an amount of current below the stimulation threshold of cardiac tissue. For example, at 5kHz, 50μA can be safely used in the cardiac chamber. As an alternative to driving current between electrodes, a sinusoidal or square wave voltage can be driven between electrodes, and current can be measured rather than potential. The signal can be generated as current driven between various sets of electrodes on the catheter 104. For example, while collecting corresponding data in response to current on other driven and passive electrodes, the signal can be generated as the current driven (i) between conduction components 150, 152; (ii) between ablation electrode 120 and the return electrode 118; or (iii) between any of the aforementioned electrodes and another internal or cutaneous electrode (not shown). Passive electrodes are electrodes on the catheter 104 that measure signals resulting from signals driven on other electrodes. It should be appreciated that the same electrode can be considered active at a given time instant or frequency and passive at others.

In order to drive multiple sets of electrodes and generate corresponding measurements on passive electrodes, various multiplexing schemes such as time division, frequency division, code division, and/or any combination thereof can be used. The current source and acquisition hardware can be implemented using discrete analog circuitry to modulate and demodulate the signals, and can also use digital-to-analog and analog-to-digital conversion to perform some of these tasks digitally using elements such as a central processing unit, a digital signal processor, and/or a field programmable gate array.

The catheter interface unit 108 includes a user interface panel (which typically includes the display 110, indicators, and switches) to permit a user to monitor and control delivery of energy to the catheter 104 from the ablation generator 116. The catheter interface unit 108 can also include a computer interface module such as a touchscreen or keyboard and mouse. The catheter interface unit 108 can display a variety of information related to the ablation procedure. For example, the catheter interface unit 108 can display a digital readout of the actual power, voltage, and/or current being delivered; the calculated impedance (e.g., based on measured current and voltage) between the ablation electrode 120 and the return electrode 118 during the delivery of energy; the measured tissue temperature on the various sensors; the number of times the ablation generator 116 has been activated; and/or the total elapsed time during which energy has been delivered to the patient 102. In some cases, the catheter interface unit 108 displays data that represents the shape of the balloon 122 or the shape or progress of a lesion being generated by the ablation catheter 104. This information can be represented in graph form, as two-dimensional images, and/or as a three-dimensional surface representing the balloon 122. The information can also be transferred to the monitoring system 112 and/or be represented in a three-dimensional electro-anatomical context.

The irrigation pump 114 is a pumping mechanism that can be connected to an irrigation fluid reservoir and operated to cause irrigation fluid (e.g., saline) to flow through the catheter 104 and exit the balloon 122. In some cases, the irrigation pump 114 contains the irrigation fluid reservoir for storing the saline, while in other cases, the irrigation pump 114 can be connected to a separate source of irrigation fluid, such as a saline bag. The irrigation pump 114 can, for example, be a peristaltic pump.

The monitoring system 112 includes a signal processor that can be connected to the sensing electrodes 146 (Figures 2-6) of the ablation catheter 104 to detect electrical activity of the heart. By sensing low frequency signals, such as below 500Hz, the electrical activity of the heart can be sensed using one or more of the pairs of concentric sensing electrodes (e.g., conductive components 150, 152 shown in Figure 5). Signals detected by the sensing electrodes 146 can also be used to generate unipolar and bipolar signals or electrograms. The electrical activity of the heart as sensed by the sensing electrodes 146 can be evaluated to analyze an arrhythmia, to determine where to deliver the ablation energy as a therapy for the arrhythmia, and to determine whether a lesion has formed in the cardiac tissue as a result of the application of the ablation energy.

As discussed above, the return electrode 118 can be separately connected to the ablation generator 116 via the cable 117. The return electrode 118 is configured for attachment to a patient's skin surface to complete the circuit loop necessary for the application of energy to the tissue of the patient 102. The return electrode 118 can be attached to the back of a patient 102. The return electrode 118 can be two or more electrodes that are conductively coupled together. It should be noted that when a large amount of power is used, more than one return electrode 118 can be used in order to limit current density at the skin. During treatment, RF power that is supplied by the ablation generator 116 is transmitted through tissue of the patient 102, between the ablation electrode 120 in the balloon 122 of the catheter 104 and the return electrode 118, to heat the tissue in the immediate vicinity of the balloon 122 to a temperature sufficient to cause ablation. The heating of the tissue can be controlled by the catheter interface unit 108 or ablation generator 116 by controlling the amount of power or current generated by the ablation generator 116. As a precautionary measure, a controller can be used to terminate heating if values or the rate of change in values of impedance, tissue temperature, lesion size, and/or other detected values, exceed predetermined safety parameters.

A method of using the ablation system 100 to perform a cardiac ablation treatment, which are not covered by the claimed invention, will now be described. Depending on the specific arrhythmia, the location or region of tissue targeted for ablation can be anatomically guided or determined by monitoring the relevant chamber or chambers of interest. Subsequently, those regions of cardiac tissue can be ablated in the manner described below. In some cases, the ablation catheter 104 or a monitoring catheter performs monitoring functions before and/or during an ablation treatment.

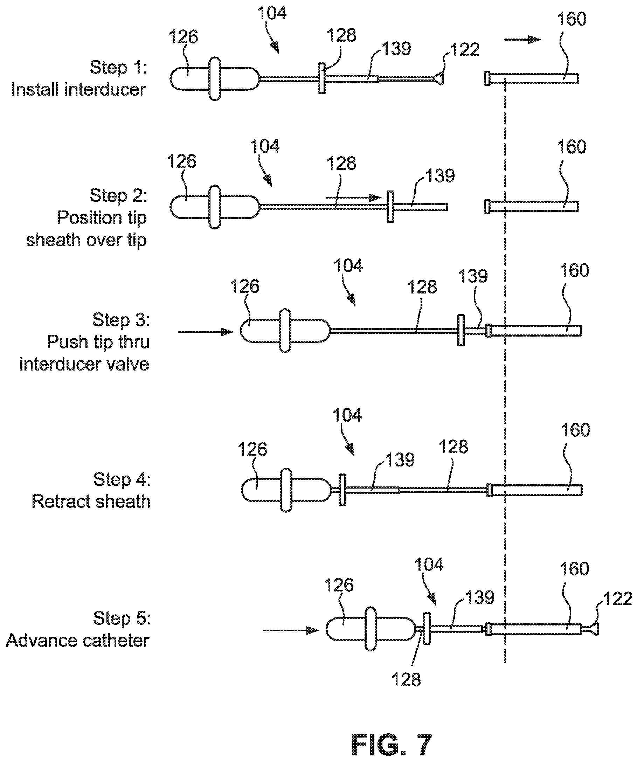

To perform a cardiac ablation treatment, the distal end of the ablation catheter 104 is first introduced into the patient 102, typically through an incision and advanced via the femoral vein or an artery. As described in greater detail below with respect to Figure 7, to facilitate insertion of the ablation catheter 104, the catheter 104 can be configured with a sheath (e.g., an insertion sheath) 139 that can be positioned around the distal end portion of the catheter shaft 128 and the balloon 122 to constrain the balloon 122 in a collapsed position. A similar sheath can be configured and inserted to predictably collapse the balloon 122 prior to extraction of the catheter 104. Constraining the balloon 122 in a collapsed configuration permits the balloon 122 to be inserted into a patient 102 via an introducer sheath 160 prior to carrying out an ablation treatment, as discussed in greater detail below. After being used to position the collapsed balloon 122 within the introducer sheath 160 and advanced through patient vasculature, the insertion sheath 139 can be proximally retracted from the balloon 122.

It has been found that such a construction allows the balloon 122 to be advanced and retracted through an introducer sheath 160, as the access point to patient vasculature. Irrigative flow is made possible through the irrigation orifices 124 in both the retracted state, as well as the normal conditions of use. In order to facilitate the collapse of the balloon 122 into a predictable shape for delivery and extraction, the balloon 122 can be configured with fine structural features. For example, the shape of the balloon 122 can include pleats formed on the neck 130 of the balloon 122. In the case where a thermoplastic is used, local heating along lines at the neck 130 can also be used to this effect. In an alternative implementation, the catheter 104 can be retracted with a slight rotation to form a twisted collapsed structure.

Figure 7 schematically illustrates a series of steps carried out to introduce the ablation catheter 104 into the patient 102. In step 1, the introducer sheath 160 is positioned within a blood vessel of the patient (e.g., the femoral artery or vein of the patient) and the ablation catheter 104 is positioned for insertion into the introducer sheath 160. As a second step, the user grasps the handle 126 of the catheter 104 and distally advances the insertion sheath 139 along the catheter shaft 128 until the insertion sheath 139 surrounds the balloon 122. As the insertion sheath 139 is advanced over the balloon 122, the balloon 122 collapses to a diameter capable of being inserted into the introducer sheath 160. The user then, in step 3, inserts the insertion sheath 139 containing the balloon 122 into the introducer sheath 160 and distally advances the catheter 104. In step 4, after positioning the balloon 122 within the introducer sheath 160, the balloon 122 is advanced out of the insertion sheath 139 that is then left surrounding a proximal portion of the catheter shaft 128 throughout the remainder of the treatment. As a fifth step, the catheter 104 is advanced through the introducer sheath 160 and the patient's vasculature until the balloon 122 reaches the treatment site in the heart of the patient. As the balloon 122 is extended distally beyond the introducer sheath 160, the balloon 122 of the catheter is allowed to expand to its nominal configuration.

The advancement of the ablation catheter 104 through the patient's blood vessels and into the patient's heart is typically viewed under fluoroscopy, but a wide range of radiological imaging can be chosen situationally. While the ablation catheter 104 is being passed through the introducer sheath, through blood vessels of the patient 102, and into the patient's heart chamber, irrigation fluid can be delivered from the irrigation pump 114 (Figure 1) to the balloon 122. Fluid may optionally weep from the balloon 122 via irrigation orifices 124 (Figures 2-5) at a low rate (e.g., about 2 ml/min), e.g., during manipulation and monitoring of the catheter 104, before ablation is initiated, and/or after ablation is completed.

After being positioned in the desired region of the heart, the ablation catheter 104 is advanced into contact with the region of the tissue to be ablated. As discussed above, fluoroscopy can be used to visualize the contact between the balloon 122 of the ablation catheter 104 and the tissue of the patient 102. Due to the conformability of the balloon 122, contact with the tissue causes the balloon 122 to deform. This deformation can be clearly viewed under fluoroscopy. In implementations where the balloon 122 does not visibly deform, or maintains stiffness as a result of internal fluid pressure, deformation of other parts of the catheter shaft 128 can be observed. In some implementations, these deformations can occur on the neck 130 of the balloon 122 indicating tissue engagement. The position sensor (not shown) within the distal end portion of the catheter shaft 128, in combination with the monitoring system visualization, can also be used to assist a user in determining whether the balloon 122 is properly positioned. In addition, the amplitude of the cardiac electric signal detected by the sensing electrodes 146 can increase as the ablation electrode 120 contacts the heart tissue if it is engaging viable tissue. The user can advance the catheter 104 until the balloon 122 has deformed by an amount equal to 1/4 to 1/2 of the diameter of the balloon 122 in the undeformed state. This deformation increases the surface area of the balloon 122 that contacts the tissue and thus increases the surface area of the tissue region that will be ablated. Other methods described in this disclosure to determine the surface area of the ablation electrode 120 in contact with tissue, which are not covered by the claimed invention, can also serve to verify proper catheter placement prior to ablation initiation.

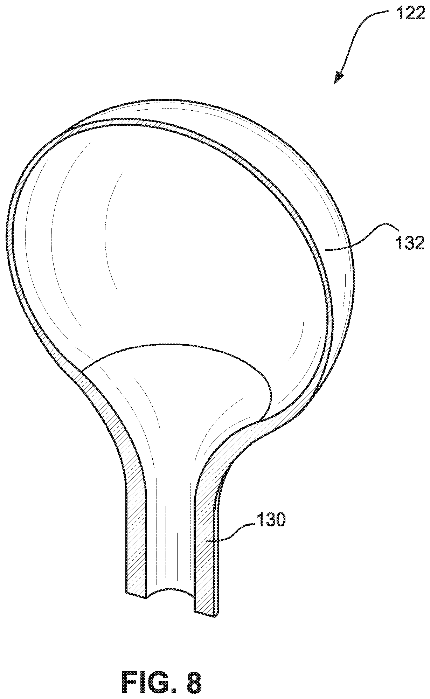

To help ensure that the contact region of the balloon 122 experiences deformation upon contacting tissue, the neck 130 and the proximal section of the body 132 can have a greater wall thickness than the distal portion of the body 132, as shown in Figure 8. The distal half of the body 132 in Figure 8 has an approximately constant wall thickness that is less than the neck 130 and the proximal half of the body 132. In other implementations, the wall thickness can increase again in the distal end region of the body 132. This can, for example, increase the ease with which the balloon 122 can be manufactured using certain manufacturing techniques (e.g., injection or blow-molding.) Without support in the proximal section of the balloon 122, a proximal axial force or a lateral force applied to the balloon 122, might cause the body 132 to shift or bend without causing a localized deformation at the distal end of the balloon 122. Specifically, the wall thickness is greatest at the neck 130 and gradually decreases up to about the mid-point (i.e., the equator) of the body 132. The thicker proximal section of the balloon 122 and neck 130 also act as a supporting structure to limit proximal movement of the proximal section of the balloon 122 in response to a proximal force being applied to the section of the balloon 122 or in response to a bending moment being applied from a lateral force in the case of side-contact. In alternative implementations described herein, the neck 130 can be deformable and can deflect as a result of tissue contact.

In one implementation, the neck 130 is configured to remain rigid to allow the distal end to absorb and conform to tissue and tissue contact. Such configurations to increase the rigidity of the catheter 104 can be useful in scenarios in which additional pressure by a user through the handle 126 is desired. The added axial and bending stiffness provided by the thickened walls at the proximal section of the balloon 122 inhibits (e.g., prevents) deformation of the proximal section of the balloon 122 resulting from these forces and thus permits localized deformation at the tissue contact region of the balloon 122.

The proximal section of the deformable balloon 122 can be supported to allow deformation of the balloon 122 to occur, without causing deformation of the neck 130 of the balloon 122. Furthermore, the neck 130 can be integrated into at least one wall of the balloon 122 or a component that is attached to the balloon 122. The balloon 122 can house one or more printed circuits 154. In one implementation, a printed circuit 154 can include a conductive material along an inner or outer surface of the deformable balloon 122. In other implementations, the printed circuit 154 includes a plurality of conductive strips that extend along the proximal section of the deformable balloon 122. The balloon 122 can include one or more internal electrodes (not shown) that can be attached to conductive wires (not shown) mounted to the flexible printed circuit 154 and arranged to transmit signals to and from the one or more internal electrodes. Further, the balloon 122 can include conductors (not shown) connected to wires of the printed circuit 154, wherein the conductors are embedded in the wall of the balloon 122 and provide an electrical connection between the wires and the outer conductive surface of the balloon 122. There can be a symmetrical arrangement of three conductors near an equator (not shown) of the balloon 122 and another three conductors in a forward hemisphere of the balloon 122.

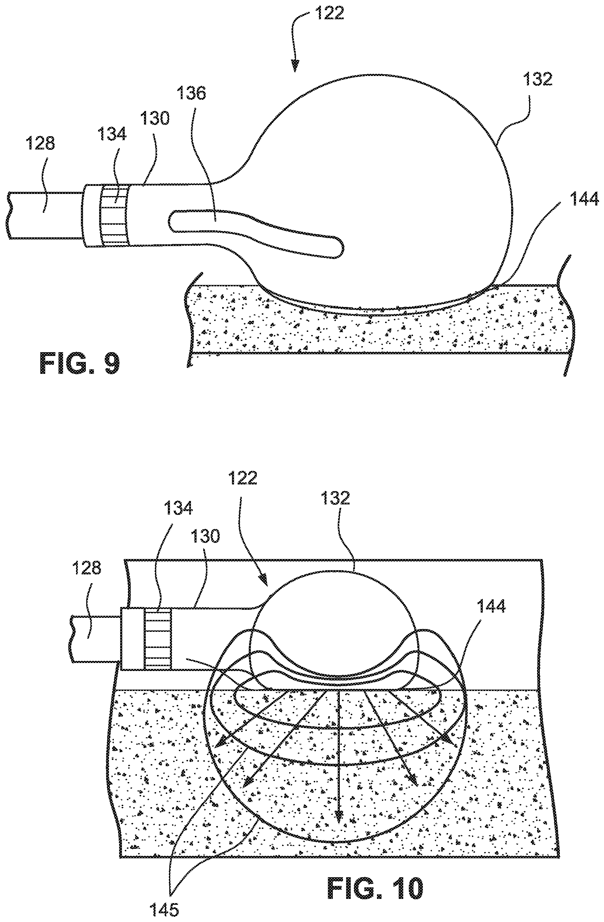

Figure 9 shows a schematic representation of the balloon 122 in lateral contact with tissue. The balloon 122 can include one or more supports 136 to maintain the shape and rigidity of the neck 130 and improve rigidity of the balloon 122 near the neck 130. Alternatives structures and/or arrangements of one or more supports 136 on the balloon 122 are shown in Figures 2 and 3. For example, the neck 130 of the balloon 122 can remain rigid so that the force of tissue contact affecting both the proximal section of the balloon 122 and the distal section of the balloon 122 preferentially results in deformation at the contact surface 144 between the balloon 122 and target tissue.

Figure 10 is a schematic representation of the balloon 122 in lateral contact with the tissue contact surface 144 during treatment. The balloon 122 is shown in a laterally deformed state and isothermal lines are illustrated to represent energy extending from the balloon 122 to the contact surface 144 and into the tissue 145.

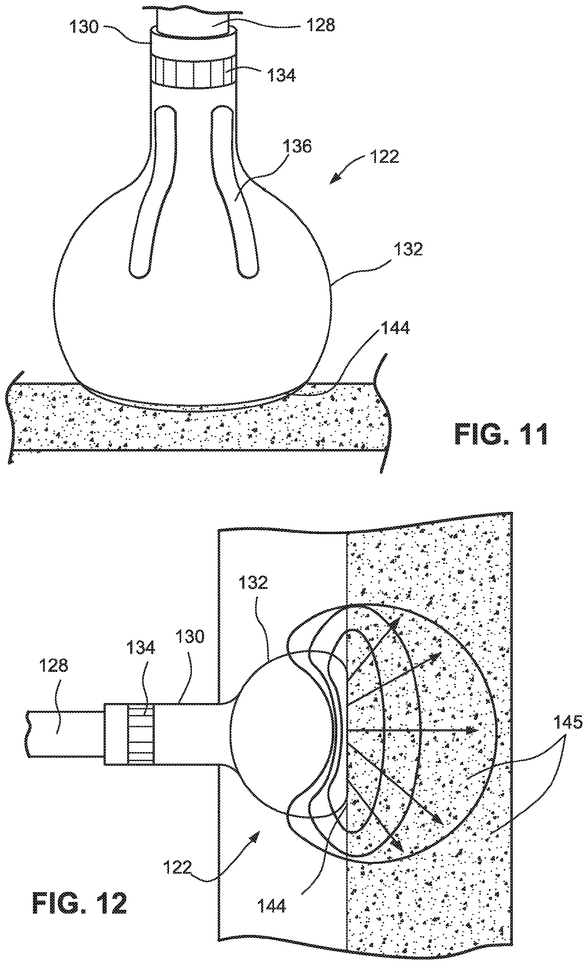

Alternatively, Figure 11 shows a schematic representation of the balloon 122 in axial contact with tissue, and Figure 12 is a schematic representation of the balloon 122 in axial contact with a tissue contact surface 144 during treatment. The balloon 122 is shown in an axially deformed state and isothermal lines are illustrated in Figure 12 to represent energy extending from the balloon 122 to the tissue contact surface 144 and into the tissue 145.

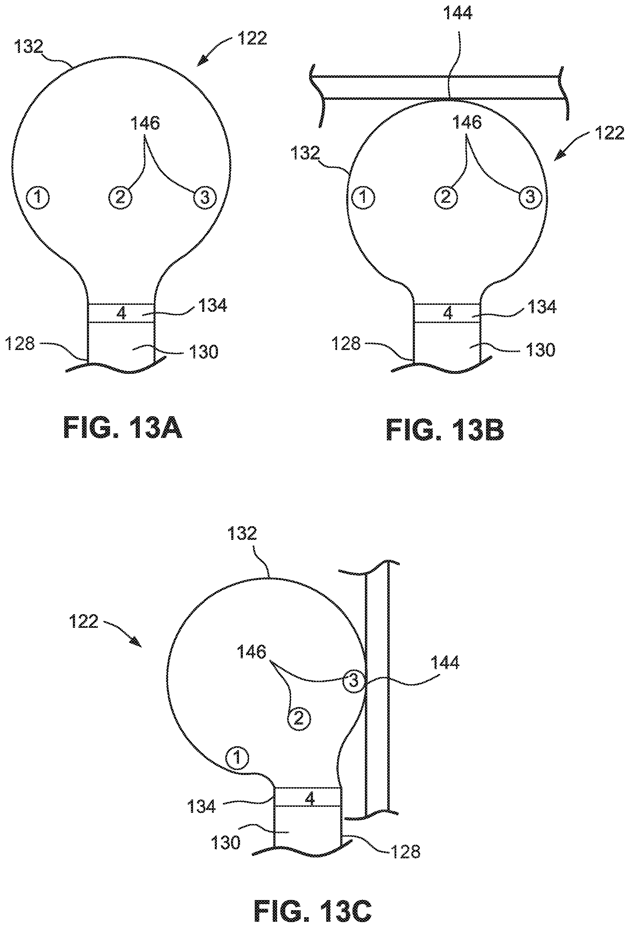

Figure 13A shows an alternative implementation in which the balloon 122 does not deform at the location of tissue contact. Detection of tissue contact is still possible in this implementation, as the force from tissue contact is applied to the balloon 122 to cause a partial deflection of the neck 130. Figure 13B shows the alternative implementation deflection in the form of compression of the neck 130 in response to tissue contact at the distal tip of the balloon 122. Figure 13C shows the alternative implementation deflection in the form of bending at the neck 130 in response to glancing or non-distal tissue contact to the balloon 122.

In alternative implementations, the deformation at the neck 130 can be viewed under fluoroscopy, and images of the deformation at the neck 130 can provide feedback on direction and extent of engagement. In addition, by driving current and detecting the impedance between sensing electrodes 146 (e.g., electrodes 1, 2, and 3) shown just below the equator of the balloon 122 and a neck-mounted electrode 134 (e.g., electrode 4) shown on the catheter shaft 128, the distance between the sensing electrodes 146 and the neck-mounted electrode 134 can be determined based on the change in impedances. It should be noted that additional schemes can be devised to enhance the accuracy and lower electrode requirements needed for shape determination.

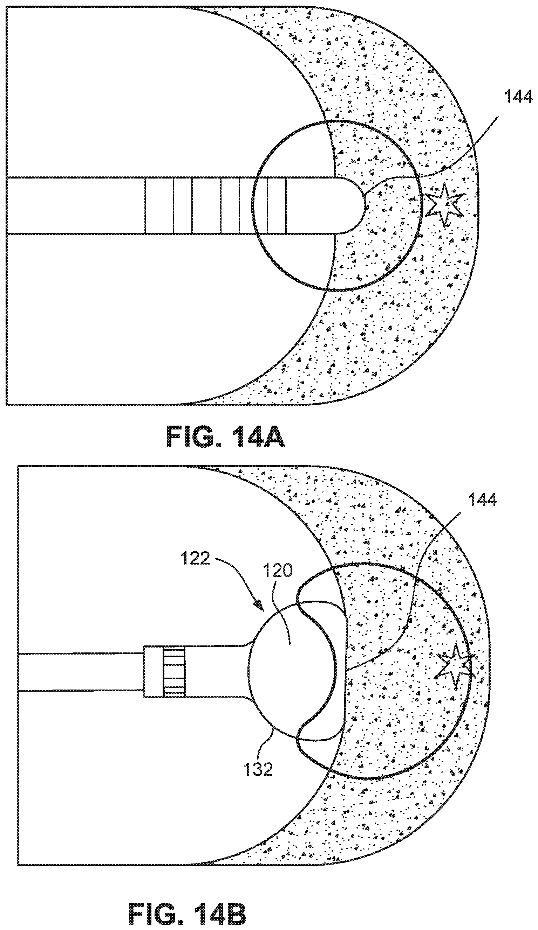

In another alternate implementation, a greater ablation depth can be achieved by a wider ablation tip. Figure 14A and 14B compare the limitation of conventional narrow tip electrodes with the wider, deformable balloon 122 that can be advantageous in treating arrhythmogenic sources that originate from deeper within cardiac tissue. While certain implementations have been described and are generally well-suited for atrial application, other implementations can be considered more suitable for deeper applications, such as forming transmural lesions in thicker ventricular tissue.

Based on sensory input, fluoroscopy, or other factors, when a user is ready to initiate an ablation process, he or she activates the ablation generator 116 (Figure 1), causing the ablation generator 116 to deliver energy to the ablation electrode 120 on the outer surface of the balloon 122. Before the ablation generator 116 is activated, the speed of the irrigation pump 114 (Figure 1) can be increased to cause irrigation fluid to flow at an increased rate (e.g., 15 ml/min). The increase of irrigation fluid flow can be preemptive or automated. The activation of the ablation generator 116 and the speed increase of the irrigation pump 114 can be triggered via an input device (e.g., button, knob, touchscreen, foot-pedal, mouse, and keyboard) of the catheter interface unit 108 (Figure 1) or of the ablation generator 116. In some implementations, a single user input can be able to initiate several functions, such as increased irrigation and/or subsequent activation of the ablation generator after a predetermined interval of time.

The irrigation pump 114 provides irrigative irrigation fluid, which is generally used to provide a cooling function at the ablation site. The irrigation fluid can cool the portions of the tissue nearest to the balloon 122. This tissue tends to absorb the most heat, but simultaneously receives the lowest cooling due to decreased blood flow. The constant supply of irrigation fluid to the portions of the tissue nearest to the balloon 122 not only helps to prevent tissue from overheating, but also helps to prevent charring and steam pop. In addition, irrigation can also play a role in preventing coagulation in blood interfacing with the ablation energy. In some implementations, an additional electrode (not shown) can be positioned inside the balloon to provide resistive heating of the irrigation fluid within the balloon 122. This arrangement can be used to control the temperature of the irrigation fluid with one of the electrodes (e.g., the proximal one) before the irrigation fluid exits the balloon 122. An internal heating electrode can be driven separately, but it also can be driven to the return electrode 118. In an alternate implementation, the irrigation fluid can be heated in the irrigation pump 114 to a first temperature that is appropriate for conduction through the catheter shaft 128. The irrigation fluid can be heated, reheated, and/or brought to the desired temperature by resistive heating at the end of the catheter shaft 128 or within the body 132 of the balloon 122. Heating at the balloon 122 provides the advantage of additional temperature control of irrigation fluid immediately before it exits the balloon 122 in contact with cardiac tissue.

In a further implementation, as the tissue is being ablated, the measurements from the sensing electrodes 146, thermistors 149, and/or internal electrodes of the balloon 122 and/or of a pressure sensor can be read by the control unit connected the catheter interface unit 108 via cable bundles (e.g., the cable 106) that extend through the catheter shaft 128. This data can be processed by the control unit, which can contain or communicate with a logic or processor, in several different ways to (i) assess a state of the balloon 122 (e.g., the degree of contact between the balloon 122 and the tissue and/or a shape of the balloon 122) and (ii) monitor the progress of the lesion being created by the energy. Alternatively, any measurements can be directly displayed to a user or practitioner.

The thermistors 149 detect the temperatures of their associated sensing electrodes 146 and transmit that temperature data to the control unit of the catheter interface unit 108. The control unit can assess the progress of the lesion being generated based on the temperature data received from the thermistors 149, including relative temperature between sensing electrodes 146.

The control unit of the catheter interface unit 108 receives the temperature data and can use that data to determine which of the sensing electrodes 146 are in contact with or in close proximity to the tissue being ablated. The sensing electrodes 146 that are in contact with or in close proximity to the tissue being ablated will detect a greater temperature than the sensing electrodes 146 that are farther away from the tissue being ablated. This is because areas exposed to blood flow lose therapeutic ablative energy through dissipative convection. Thus, the control unit, user, or practitioner can determine which regions of the balloon 122 are contacting or nearly contacting the tissue by determining which of the sensing electrodes 146 have the highest temperatures.

When a constant current is driven from the conductive outer surface (the ablation electrode 120) to the return electrode 118, each sensing electrode 146 can be passive and sense a voltage difference between its conductive components 150, 152. The voltage difference between the conductive components 150, 152 and the current applied between the conductive outer surface and the return electrode 118 can be used to determine an impedance. The conductive outer surface and the return electrode 118 can drive and measure current, and passive conductive components 150, 152 can be arranged in a four-wire sensing arrangement to detect a voltage difference representative of resistance or impedance. The sensed resistance or impedance provides a reliable indicator of the electrical condition at a local region near the pair of conductive components 150, 152 of the sensing electrode 146. The resistance or impedance can be used to detect good contact between tissue and the conductive surface near the sensing electrode 146, an undesired blood coating on the sensing electrode 146, and a poor current flow to the return electrodes 118. Each of these conditions can be cause to generate an alert and stop application of ablation energy.

Impedance values can be evaluated by the processor in the system 100 of the catheter 104 to determine the relative orientation of each of the sensing electrodes 146, in addition their proximity to tissue. Such calculations might also allow the system to determine not only tissue contact, but also the relative shape of the balloon 122. It should further be appreciated that by determining tissue contact and conductive outer surface shape, irrigation fluid volume at the balloon 122 can also be estimated. It is appreciated that the complexity of the determination can dictate that empirically determined values can provide the best estimates in practice.

In some implementations, as shown, it can be preferred that the sensing electrodes 146 are configured as concentric electrode pairs. The concentric pairs can comprise a central conductive component 150 and an annular conductive component 152 that surrounds the central conductive component 150. By driving a current independent of the ablation energy via the conductive components 150, 152, the corresponding impedance measured is based on the current and environment at that particular sensor location. Because of the proximity of the conductive components 150, 152, any tissue impedance measured across the conductive components 150, 152 provides feedback regarding tissue at that location. Therefore, tissue impedance measured between conductive components 150, 152 can more accurately determine lesion formation. Tissue impedance decreases with lesion formation. The electrical detection of reduced amplitude or phase change can imply tissue temperature fluctuations and lesion formation, as indicated by impedance change in surrounding tissue.

In some implementations, impedance is an indicator of lesion formation. Detecting lesion formation based on tissue impedance can help to ensure that lesions are sufficiently consistent. In addition, a measurement of tissue impedance can reveal if tissue has been ablated to sufficient depths or is transmural. Lesion feedback can also provide a mechanism by which the ablation application is terminated at the moment of sufficient lesion formation. Such monitoring can accurately determine when the application of ablation energy can be terminated and can help protect critical surrounding tissue from excess heating.

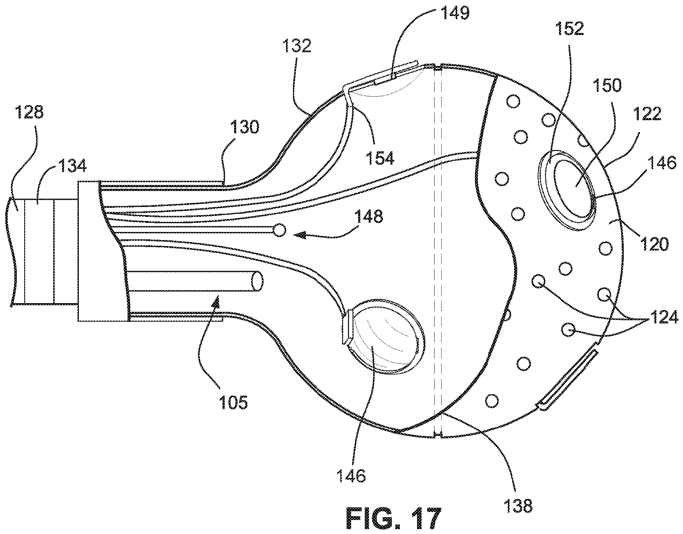

The control unit of the catheter interface unit 108 can process the above-discussed data from the thermistors 149, the internal electrodes (e.g., the internal electrode 148 shown in Figure 17), the sensing electrodes 146, and the return electrode 118 in any of the various ways discussed above to determine or approximate the shape of the balloon 122 or characteristics of the lesion created. The control unit can then generate and output signals to the display 110 of the catheter interface unit 108 that are representative of the shape of the balloon 122 or the characteristics of the lesion. The (i) shape of the balloon 122 or (ii) characteristics of the lesion or cluster of lesions can then be displayed on the display 110 of the catheter interface unit 108.

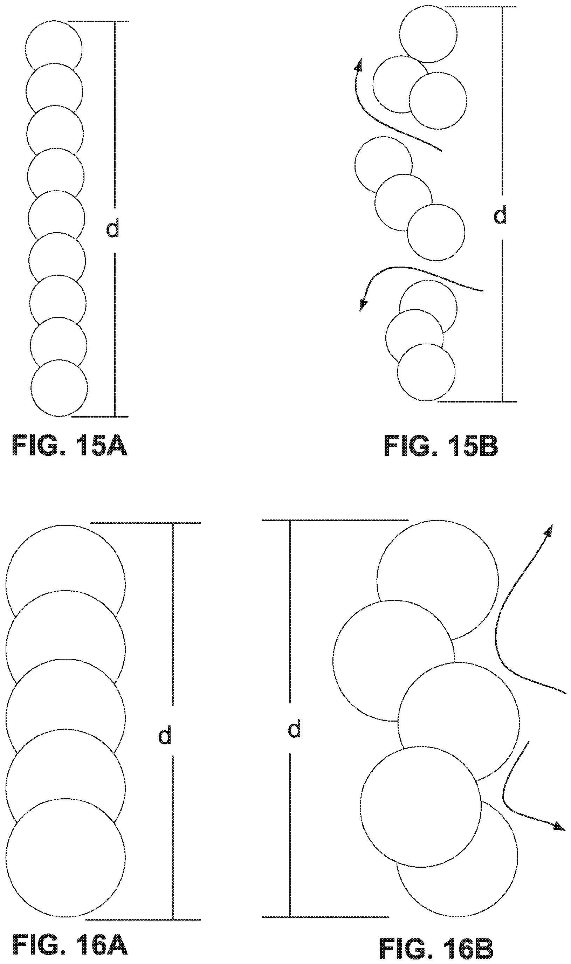

Assessment of lesion progress can assist not only in directing electrode placement before applying ablation therapy, but also in confirming the continuity of the lesion. Figures 15A-16B, for example, illustrate the importance of preventing gaps and ensuring lesion continuity for blocking electrical transmission within cardiac tissue. Figures 15A and 15B show a theoretical rendering of conventional ablation treatment. In particular, Figure 15A shows an ideal therapy with applications forming a narrow, continuous ablation pattern covering a distance d. In contrast, Figure 15B is a rendering of a possible pattern formed in vivo in which ablation applications cover a distance d, but form gaps in lesion formation due to inconsistencies in tip placement. Figures 16A and 16B show a theoretical rendering of an ablation treatment with a larger ablation tip. In particular, Figure 16A shows an ideal therapy with few applications needed to form a wide, continuous ablation pattern covering a distance d. In contrast, Figure 16B is a rendering of a possible pattern formed in vivo in which inconsistencies in tip placement change the pattern of lesion formation. Due to the increased lesion width, the ablation applications are able to cover distance d with the same number of applications and user or placement errors do not result in the formation of gaps.

The physician can use imaging to better understand the relative amount of contact the balloon 122 is making with the tissue and the size and shape of the lesion being formed. In an ideal application of therapy, as shown in Figures 15A and 16A, lesions are formed with partial overlapping to reduce the risk and effects of non-continuous lesion formation. Gaps in the therapy pattern, as shown in Figure 15B, can reduce the efficacy of the treatment in preventing arrhythmias. As shown in Figure 16B, increasing the width of lesion sizes not only reduces the total number of lesions needed to span a distance, d, but also provides additional overlap to ensure that variations in lesion formation that can arise in vivo do not adversely affect the overall success of the ablation therapy.

Since the area of contact with the endocardium is actively cooled with irrigation fluid, permanent lesions can sometimes not form on the endocardium. This effect is referred to as endocardial sparing and is undesirable when applying a permanent ablation lesion. The following describes a titration of power and flow in order to maximize lesion size and avoid endocardial sparing. In the first phase, lasting approximately 30 seconds, high (e.g., 15-30 mL/min) flow could be used with a lower power setting (e.g., 40W). This phase helps heat the immediate endocardial tissue, reducing its impedance, thus allowing deeper penetration of power in the second phase. Once heating and impedance drop is accomplished, a second phase, lasting approximately 30 seconds, with the same flow and higher power (e.g., 300W) can be applied. In this phase, the highest power and the most energy is delivered, making this phase primarily responsible for the lesion's eventual depth. Subsequently, a third phase targets endocardial tissue lasting approximately 30 seconds, where both power and flow are lowered (5-10 mL/min and 40W). Due to the lower flow, the endocardium receives less cooling and is more likely to be included in the permanent lesion. In order to avoid steam pop and char formation, power is also lowered in this third phase. The transition between the phases can be done suddenly (e.g., in a single step) or gradual (e.g., over a short period of time). This sequence in its entirety can be programmed in advance into the ablation generator 116 or catheter interface unit 108 and implemented by the system 100. Alternatively, this sequence can be manually implemented by hospital staff as the lesion is delivered. While specific time periods, power settings, and flow settings have been described for the ablation phases, others could be used.

Figure 17 shows a partial cross-sectional view of a balloon 122 of the ablation catheter 104 of Figure 2, with the open, cross-sectional area showing the interior of a catheter shaft 128 defining a fluid delivery lumen 105. As shown, the catheter 104 includes an internal sensing electrode 148; a printed circuit 154; irrigative orifices, holes, or apertures 124; ablation electrode 120, thermistors 149, sensing electrodes 146 having conductive components 150, 152; and other electrodes.

While the thermistors 149 of the balloon 122 have been described as being attached to portions of the flexible printed circuit 154 opposite the central conductive components 150 of the various sensing electrodes 146, the thermistors 149 can alternatively or additionally be attached to portions of the flexible printed circuit 154 opposite the annular conductive components 152 of the sensing electrodes 146. The thermistors 149 can alternatively be directly connected to one of the conductive components 150, 152 of the sensing electrodes 146 (e.g., via openings formed in the flexible printed circuit 154). In addition, while the inner electrodes and the sensing electrodes 146 and, in some cases, the thermistors 149 have been described as being attached to discrete flexible circuit pads, in certain implementations, the inner electrodes, the sensing electrodes 146, and the thermistors 149 are attached to conductive strips of the flexible printed circuit 154 that extend from the catheter shaft 128 along the balloon 122. These strips could themselves be discrete elements (e.g., separate printed circuits 154), or branches of a single flexible circuit 154.

Various forms of thermistors 149 and thermocouples can be implemented. Thermistors 149 and thermocouples can be applied to the flexible printed circuit 154 as thin film or ink applied as part of the flexible printed circuit manufacturing process. Furthermore, rather than on the flexible printed circuit 154, these thermistors 149 and thermocouples can be applied directly to the balloon 122. For example, the thermistors 149 and thermocouples could be formed directly using appropriate thermistor or conductive materials, e.g., conductive inks, on the balloon 122.

While sensing electrodes 146 have generally been described as circular or concentric, other shapes can be used or relative locations can be used. For example, the sensing electrodes 146 could be oval or rectilinear, or positioned in close, but not concentric, proximity. In some examples, the sensing electrodes 146 can be individual electrodes or individual parts (e.g., pieces of metal) assembled on the balloon 122. Conductive components 150, 152 can form full or partial circles, as long as they are substantially concentric with respect to each other.

While certain balloons 122 described above in at least one implementation have generally smooth outer surfaces, the balloon 122 could be textured, dimensioned to improve irrigative flow, or configured to improve tissue contact. Such a design can help to ensure that irrigation fluid exiting the irrigation orifices 124 is able to flow out of the balloon 122 and along the tissue by preventing the tissue from forming a complete seal with the outer surface regions. Irrigation orifices 124 are connected with a network of channels guaranteeing that fluid irrigation is still active in regions where the balloon 122 is pressed against tissue.

Other techniques can also be used to help prevent fluid stasis in the region of tissue contact. One further balloon 122 includes projections that extend outwardly from regions of the outer surface between adjacent recessed regions. These projections cause separation between the patient's tissue and portions of the outer surface of the balloon 122, which form the irrigation orifices 124. As a result, the balloon 122 of this implementation can help to ensure that irrigation fluid is able to exit the irrigation orifices 124 and flow between the outer surface of the balloon 122 and the patient tissue as opposed to stagnating. The projections can also increase friction between the balloon 122 and the cardiac tissue by lodging in rugged surfaces such as trabeculations, leading to improved mechanical stability and reduced movement of the catheter 104 during ablation delivery.

In an additional implementation, a balloon 122 can be configured to include a conical support extending between a neck 130 of the balloon 122 and a proximal section of a body 132 of the balloon 122. The conical structure can be a solid structure that acts much like a thickened wall region of the balloon 122. Alternatively, the conical support can be a hollow member, which can reduce the total amount of material required to form the balloon 122. In either case, the conical support can be either integrally formed with the neck 130 and body 132 of the balloon 122 or can be separately formed and then attached (e.g., thermally or adhesively bonded) to the neck 130 and body 132 of the balloon 122.

In a further implementation, a balloon 122 can be configured to include multiple ribs that extend along the outer surface of the proximal section of a body 132 of the balloon 122. In certain implementations, the ribs are formed of the same material as the body 132 of the balloon 122. In such implementations, the increased thickness of that material in the regions including the ribs provide the proximal section of the balloon 122 with increased rigidity. In other implementations, the ribs are formed of harder materials and are attached (e.g., thermally, adhesively bonded or molded) to the body 132 of the balloon 122. The use of harder materials to form the ribs can ensure that sufficient rigidity is imparted to the proximal section of the balloon 122 while limiting the overall thickness of the proximal section of the balloon 122.

The body 132 can also include fold lines, such as longitudinal lines at which the thickness of the body 132 is thinner as compared to other portions of the body 132. The fold lines allow the body 132 to collapse in a predetermined manner. The collapsed body 132 can be inserted into a sheath of a catheter system 100.

In a further implementation, the material of the balloon 122 is applied to the flexible printed circuit material in multiple discrete segments that can increase in width as they extend away from the flexible printed circuit material to provide asymmetrical bending behaviors. The material of the balloon 122 is dimensioned to form any of various shapes that are wider along a top edge than along a bottom edge. This arrangement allows for a radial inward force (i.e., a downward force in the illustrated view) that is applied to the proximal section of the balloon 122 (i.e., the region of the balloon 122 along which the conductive strips of the flexible printed circuit 154 and stiffening segments extend) to inwardly deflect the proximal section of the balloon 122. As a result of this inward force, the discrete segments of stiffening material will be spaced apart. In contrast, the discrete segments of stiffening material will limit (e.g., prevent) the proximal section of the balloon 122 from deflecting outward (e.g., in response to a proximal force applied to the distal end region of the balloon 122). Such a force would compress the discrete segments of stiffening material together. Due to the close proximity of the neighboring discrete segments and the stiffness of those segments, very little movement in the radial outward direction will be allowed. In some cases, as few as one functionally equivalent shape and one rigid element near the neck 130 provides asymmetric bending behavior.

While the ablation electrode 120 has been described in the form of a continuous conductive layer applied to the outer surface of the balloon 122, any of various other patterns can alternatively be used. For example, the ablation electrode 120 can extend at least partially between each of the plurality of sensing electrodes 146 and each of the other sensing electrodes 146 of the plurality of sensing electrodes 146. As used herein, the at least partial extension of the ablation electrode 120 between two sensing electrodes 146 can include a pattern in which, for example, the ablation electrode 120 is discontinuous along a circumferential segment between the two sensing electrodes 146. Further, or instead, the at least partial extension of the ablation electrode 120 between two sensing electrodes 146 can include a pattern in which the ablation electrode 120 is continuous along a circumferential segment between the two sensing electrodes 146.

While the above-described balloons 122 include ablation electrodes 120 in the form of conductive layers, other types of ablation electrodes 120 can alternatively or additionally be used with any of those balloons 122. In certain implementations, for example, the material of the body 132 and the neck 130 of the balloon 122 is loaded with an electrically conductive material such that material of the body 132 and the neck 130 can transmit energy to irrigation fluid within the balloon 122. In certain implementations, for example, the balloon 122 is formed of a polymer that is loaded with electrically conductive particles. Examples of suitable polymers include, for example, silicone, SEBS, polyurethane, nylon, and PEBAX. Examples of suitable electrically conductive particles include, for example, silver particles, carbon particles, and gold particles.

While many of the balloons 122 discussed above have been described as including thermistors 149, other types of temperature sensors, such as thermocouples, can alternatively or additionally be used. For example, ink-based thermocouple can be applied directly to outer surface of balloon 122, ink-based thermistors, thin-film elements, e.g., ink-on-flex circuits assembled on balloon 122, and other temperature sensors can be used.

While the balloons 122 discussed above have been described as having a spherical shape, balloons or expandable elements 122 having other shapes can be used. The shape of the balloon 122 can, for example, be selected to correspond to the shape of an anatomical feature to be treated or to perform a particular type of procedure desired. Other balloons 122 can have tear drop and garlic clove shapes, respectively. The distal section of the body 132 of each of these balloons 122 is generally rounded or hemispherical and the proximal section tapers to a smaller diameter toward the proximal end. Such a balloon shape can provide more structural support in the proximal section while maintaining a large surface area in the distal section, which allows for the formation of large lesions. In certain implementations, a balloon 122 having a football shape or hammerhead shark shape can be used. This type of balloon 122 can be particularly beneficial when ablation along a line of contact with tissue is desired. In a further implementation, an opposed-cones shaped balloon 122, which can be used to make a lesion in the form of a circle when the deformable balloon 122 is pressed into an opening (e.g., of a vein) of arbitrary diameter. Any of various other asymmetric shapes can alternatively be implemented in order to selectively ablate in one area, or to fit the balloon 122 into a particularly shaped anatomical structure.

While the balloon 122 has generally been described as being loaded with a radiopaque additive such as a barium sulfate or bismuth additive, other materials or inks could be used to permit the balloon 122 to be visualized under fluoroscopy, during an ablation treatment. In some implementations, a stiffening material, e.g., Nitinol, can be added to the balloon 122 to facilitate visualization during an ablation treatment.

The distal section of the catheter shaft 128 can be implemented as a single lumen. In other implementations, the catheter shaft 128 includes two or more lumens. In certain implementations, for example, the catheter shaft 128 includes four lumens for separately housing the various wires and pull cables and tube transporting the irrigation fluid.

While the ablation catheter 104 has been described as a bi-directional, steerable catheter 104, the catheter 104 can alternatively be a unidirectional, steerable catheter 104. For example, rather than having two wires that are circumferentially spaced by about 180 degrees and are attached to the ring positioned at the distal end of the catheter shaft 128, a single wire can extend along the catheter shaft 128 and be attached to the ring. In addition, while the catheter 104 has been described as including a deflectable or steerable catheter shaft 128, non-steerable catheter shafts 128 can alternatively be used in combination with or independently from a fixed-curve or articulating sheath, guidewire or robotic navigation systems.

While the ablation catheter 104 has been described as including an insertion sheath 139 that is retractable for constraining the balloon 122 in a collapsed configuration, other types of sheaths can be used. In some implementations, for example, the sheath 139 is designed to be removable from the catheter shaft 128 after insertion of the balloon 122 into the patient 102. In this example, the torn sheath 139 can be removed from the catheter shaft 128.

While the balloons 122 of many ablation catheters 104 described herein have been described as expanding to a larger size after delivery into the patient 102, in some implementations a balloon 122 of an ablation catheter 104 can expand to a larger size after being delivered to the ablation site.

While the ablation catheters 104 above have been described as including a sheath 139 to constrain the balloon in a collapsed configuration while the ablation catheter is being inserted through an introducer sheath 160 into the patient, other types of devices can alternatively or additionally be used to constrain the balloon 122 in its collapsed configuration. In some implementations, for example, the ablation catheter 104 includes an axially displaceable rod positioned within an irrigation lumen 105 defined by the catheter shaft 128. The distal end of the rod typically terminates in or near the balloon 122. In certain implementations, the distal end of the rod is attached to (e.g., mechanically attached to or adhesively bonded to) the inner surface of the distal end of the balloon 122. The distal end of the rod can include a bulbous head or some other type of blunt feature to help prevent damage to the balloon 122 due to contact between the distal end of the rod and the inner surface of the balloon 122. The proximal end of the rod extends proximally from the proximal end of the handle 126 such that the user is able to grasp and manipulate the portion of the rod proximal to the handle 126. Prior to insertion of the ablation catheter 104 into the patient 102, the user can push the rod in the distal direction. When doing so, the distal end of the rod contacts the inner surface of the distal end of the balloon 122, causing the balloon 122 to lengthen and partially collapse. This technique reduces the overall diameter of the balloon 122, allowing it to be more easily inserted into the patient 102.

While measured or monitored values have generally been described as impedance values, other values, or parameters can be used. For example, voltage values and current values, and so forth can be additionally or alternatively be measured or monitored.

While data collected from the various sensors has been described as being sent to the catheter interface unit 108 or control unit for processing, the data can alternatively be sent to other devices for processing. In certain implementations, for example, data is sent from sensors of the ablation catheter 104 to the monitoring system 112 or recording system 111.

While the catheter interface unit 108, the recording system 111, the monitoring system 112, the irrigation pump 114, and the ablation generator 116 have been described as separate components, in certain implementations, two or more of those components are integrated into a single machine. In some implementations, for example, the catheter interface unit 108 is integrated with the ablation generator 116 and the irrigation pump 114. In certain implementations, all of these components are integrated into a single machine.

While the systems above have been described as using saline 123, any of various other biocompatible electrically conductive fluids can alternatively or additionally be used. In some examples, the saline is a hypertonic saline allowing improved energy delivery, e.g., through lower impedance.

While the systems and methods described above relate to RF ablation, which are not covered by the claimed invention, in certain implementations other types of energy can be used with these systems and methods. For example, irreversible electroporation includes a sequence of brief but high voltage energy application to induce tissue apoptosis and form a lesion. One of the challenges associated with irreversible electroporation includes energy loss during energy delivery to a target tissue. Just as with RF ablation, the systems and methods described herein, which are not covered by the claimed invention, are also particularly well suited for irreversible electroporation due to the relatively large conductive outer surface-tissue contact area, and the ability to know catheter state prior to energy application allows selective application of the electroporation energy to target tissue. One added benefit of electroporation is the notable reduction in convective heating from the application of a high voltage pulse or series of pulses. Alternatively, however, the larger balloon 122 provides the option of deeper lesions, as well, due to the creation of a larger and penetrating electrical field. Control of the increase variety of lesion sizes and depths provide users with the specificity needed to treat a range of cardiac ablation needs.

While the ablation catheter 104 has been generally described as being used to perform a cardiac ablation treatment, the ablation catheter 104 can alternatively or additionally be used to carry out various other types of treatments. Applied at longer duration under greater power, compared to conventional tips, the catheter 104 described herein facilitates deeper lesion formation and can also be effective for performing ventricular ablations, as the muscles of the ventricle are deeper than those found in the atria. Other treatments include, but are not limited to: the ablation of tumors, the ablation of uterine tissue to control excessive uterine bleeding, renal and carotid denervation, and biological agent delivery.

D. Conclusion

The above detailed descriptions of implementations of the technology are not intended to be exhaustive or to limit the technology to the precise form disclosed above. Although specific implementations of, and example for, the technology are described above for illustrative purposes, various modifications are possible within the scope of the technology as defined by the appended claims. For example, while steps are presented in a given order, alternative implementations may perform steps in a different order. Furthermore, the various implementations described herein may also be combined to provide further implementations.

From the foregoing, it will be appreciated that specific implementations of the technology have been described herein for purposes of illustration, but well-known structures and functions have not been shown or described in detail to avoid unnecessarily obscuring the description of the implementations of the technology. Where the context permits, singular or plural terms may also include the plural or singular term, respectively. Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Where the context permits, singular or plural terms may also include the plural or singular term, respectively. Additionally, the terms "comprising," "including," "having" and "with" are used throughout to mean including at least the recited feature(s) such that any greater number of the same feature and/or additional types of other features are not precluded..

From the foregoing, it will also be appreciated that various modifications may be made without deviating from the technology as defined by the appended claims. For example, various components of the technology can be further divided into subcomponents, or that various components and functions of the technology may be combined and/or integrated. Furthermore, although advantages associated with certain implementations of the technology have been described in the context of those implementations, other implementations may also exhibit such advantages, and not all implementations need necessarily exhibit such advantages to fall within the scope of the invention defined by the appended claims.

**Claims**

1. A catheter (104), comprising:

   a catheter shaft (128) having a proximal end portion and a distal end portion, the catheter shaft defining an irrigation lumen;
   a balloon (122) including a proximal section and a distal section, wherein the proximal section of the balloon is coupled to the distal end portion of the catheter shaft (128), and wherein the balloon defines a volume in fluid communication with the irrigation lumen (105), and further wherein the balloon defines a plurality of irrigation orifices (124) in fluid communication with the volume;
   an ablation electrode (120) disposed along at least the distal section of the balloon (122); wherein the ablation electrode (120) includes a continuous conductive outer layer on an outer surface of the distal section of the balloon (122); and
   a plurality of sensing electrodes (146) disposed along at least the distal section of the balloon (122), wherein each sensing electrode (146) is electrically isolated from the ablation electrode (120), and wherein each sensing electrode (146) is bounded by the ablation electrode (120), wherein, independent of an orientation of the distal section of the balloon to tissue, any line of contact between the distal section of the balloon and tissue is substantially continuously spanned by the ablation electrode, one or more of the sensing electrodes, or a combination thereof.

2. The catheter of claim 1 wherein, along an outer surface of the balloon (122), the ablation electrode (120) extends at least partially between each sensing electrode (146) of the plurality of sensing electrodes and each of the other sensing electrodes (146) of the plurality of sensing electrodes.

3. The catheter of claim 1 or claim 2, wherein the ablation electrode (120) extends continuously between a first sensing electrode (146) of the plurality of sensing electrodes and a second sensing electrode (146) of the plurality of sensing electrodes.

4. The catheter of any one of claims 1-3, wherein the volume defined by the balloon (122) is in thermal communication with the ablation electrode (120).

5. The catheter of any one of claims 1-4, wherein at least the distal section of the balloon (122) is semi-compliant.

6. The catheter of claim 5 wherein, in response to an axial force, the distal section of the balloon (122) is more compliant than the proximal section of the balloon (122).

7. The catheter of claim 6 wherein the balloon (122) includes a neck (130) disposed along the proximal section of the balloon (122).

8. The catheter of claim 7 wherein the balloon (122) includes a self-expandable support structure configured to expand the balloon (122) from a collapsed state to an expanded state.

9. The catheter of any one of claims 1-8, wherein at least some irrigation orifices (124) of the plurality of irrigation orifices extend through the ablation electrode (120).

10. The catheter of any one of claims 1-9, wherein at least some irrigation orifices (124) of the plurality of irrigation orifices are positioned to direct fluid in a proximal direction relative to the balloon (122).

11. The catheter of any one of claims 1-10 wherein the unitary conductive outer layer includes a conductive ink disposed on the outer surface of the balloon (122) or a polymer loaded with electrically conductive particles.

12. The catheter of any one of claims 1-11, wherein the balloon (122) is inflatable from a collapsed state to an expanded state, and wherein, in the expanded state of the balloon (122), the ablation electrode (120) extends along at least a distalmost point of the balloon (122) in the absence of a force on an external surface of the balloon (122).

13. The catheter of any one of claims 1-12, further comprising a plurality of temperature sensors disposed along the balloon (122), wherein each temperature sensor is thermally isolated from irrigation fluid in the volume defined by the balloon (122).

14. The catheter of claim 13, wherein at least some of the temperature sensors of the plurality of temperature sensors are disposed along the distal section of the balloon (122).

15. The catheter of one of claims 1-14, further comprising flexible printed circuits (154) disposed along the balloon (122), and wherein each sensing electrode of the plurality of sensing electrodes (154) is formed along a respective flexible printed circuit (154).

16. The catheter of claim 1, wherein the conductive outer layer covers greater than 90% of the outer surface of the balloon.

**Patentansprüche**

1. Katheter (104), umfassend:

einen Katheterschaft (128), der einen proximalen Endabschnitt und einen distalen Endabschnitt aufweist, wobei der Katheterschaft ein Spüllumen definiert;
einen Ballon (122), der einen proximalen Bereich und einen distalen Bereich einschließt, wobei der proximale Bereich des Ballons mit dem distalen Endabschnitt des Katheterschafts (128) gekoppelt ist, und wobei der Ballon ein Volumen in Fluidverbindung mit dem Spüllumen (105) definiert, und ferner wobei der Ballon eine Vielzahl von Spülöffnungen (124) in Fluidverbindung mit dem Volumen definiert;
eine Ablationselektrode (120), die entlang mindestens des distalen Bereichs des Ballons (122) angeordnet ist; wobei die Ablationselektrode (120) eine durchgehende leitfähige Außenschicht auf einer Außenoberfläche des distalen Bereichs des Ballons (122) einschließt; und
eine Vielzahl von Erfassungselektroden (146), die entlang mindestens des distalen Bereichs des Ballons (122) angeordnet sind, wobei jede Erfassungselektrode (146) von der Ablationselektrode (120) elektrisch isoliert ist, und wobei jede Erfassungselektrode (146) durch die Ablationselektrode (120) begrenzt ist,
wobei, unabhängig von einer Ausrichtung des distalen Bereichs des Ballons zu Gewebe, eine beliebige Kontaktlinie zwischen dem distalen Bereich des Ballons und dem Gewebe im Wesentlichen durch die Ablationselektrode, eine oder mehrere der Erfassungselektroden oder eine Kombination davon durchgehend gespannt ist.

2. Katheter nach Anspruch 1, wobei sich die Ablationselektrode (120) entlang einer Außenoberfläche des Ballons (122) mindestens teilweise zwischen jeder Erfassungselektrode (146) der Vielzahl von Erfassungselektroden und jeder der anderen Erfassungselektroden (146) der Vielzahl von Erfassungselektroden erstreckt.

3. Katheter nach Anspruch 1 oder 2, wobei sich die Ablationselektrode (120) zwischen einer ersten Erfassungselektrode (146) der Vielzahl von Erfassungselektroden und einer zweiten Erfassungselektrode (146) der Vielzahl von Erfassungselektroden durchgehend erstreckt.

4. Katheter nach einem der Ansprüche 1 bis 3, wobei das Volumen, das durch den Ballon (122) definiert ist, in thermischer Verbindung mit der Ablationselektrode (120) steht.

5. Katheter nach einem der Ansprüche 1 bis 4, wobei mindestens der distale Bereich des Ballons (122) halbnachgiebig ist.

6. Katheter nach Anspruch 5, wobei als Reaktion auf eine axiale Kraft der distale Bereich des Ballons (122) nachgiebiger als der proximale Bereich des Ballons (122) ist.

7. Katheter nach Anspruch 6, wobei der Ballon (122) einen Hals (130) einschließt, der entlang des proximalen Bereichs des Ballons (122) angeordnet ist.

8. Katheter nach Anspruch 7, wobei der Ballon (122) eine selbstexpandierbare Stützstruktur einschließt, die konfiguriert ist, um den Ballon (122) von einem zusammengefallenen Zustand in einen expandierten Zustand zu expandieren.

9. Katheter nach einem der Ansprüche 1 bis 8, wobei sich mindestens einige Spülöffnungen (124) der Vielzahl von Spülöffnungen durch die Ablationselektrode (120) erstrecken.

10. Katheter nach einem der Ansprüche 1 bis 9, wobei mindestens einige Spülöffnungen (124) der Vielzahl von Spülöffnungen positioniert sind, um Fluid in einer proximalen Richtung relativ zu dem Ballon (122) zu leiten.

11. Katheter nach einem der Ansprüche 1 bis 10, wobei die einheitliche leitfähige Außenschicht eine leitfähige Tinte, die auf der Außenoberfläche des Ballons (122) angeordnet ist, oder ein Polymer einschließt, das mit elektrisch leitfähigen Partikeln beladen ist.

12. Katheter nach einem der Ansprüche 1 bis 11, wobei der Ballon (122) von einem zusammengefallenen Zustand in einen expandierten Zustand aufblasbar ist und wobei sich die Ablationselektrode (120) in dem expandierten Zustand des Ballons (122) entlang mindestens eines distalsten Punkts des Ballons (122) in Abwesenheit einer Kraft auf eine äußere Oberfläche des Ballons (122) erstreckt.

13. Katheter nach einem der Ansprüche 1 bis 12, ferner umfassend eine Vielzahl von Temperatursensoren, die entlang des Ballons (122) angeordnet sind, wobei jeder Temperatursensor von Spülfluid in dem Volumen, das durch den Ballon (122) definiert ist, thermisch isoliert ist.

14. Katheter nach Anspruch 13, wobei mindestens einige der Temperatursensoren der Vielzahl von Temperatursensoren entlang des distalen Bereichs des Ballons (122) angeordnet sind.

15. Katheter nach einem der Ansprüche 1 bis 14, ferner umfassend flexible Leiterplatten (154), die entlang des Ballons (122) angeordnet sind, und wobei jede Erfassungselektrode der Vielzahl von Erfassungselektroden (154) entlang einer jeweiligen flexiblen Leiterplatte (154) ausgebildet ist.

16. Katheter nach Anspruch 1, wobei die leitfähige Außenschicht mehr als 90 % der Außenoberfläche des Ballons bedeckt.

## Revendications

1. Cathéter (104) comprenant :

une tige de cathéter (128) ayant une partie d'extrémité proximale et une partie d'extrémité distale, la tige de cathéter définissant une lumière d'irrigation ;
un ballonnet (122) comportant une section proximale et une section distale, dans lequel la section proximale du ballonnet est accouplée à la partie d'extrémité distale de la tige de cathéter (128), et dans lequel le ballonnet définit un volume en communication fluidique avec la lumière d'irrigation (105), et dans lequel le ballonnet définit en outre une pluralité d'orifices d'irrigation (124) en communication fluidique avec le volume ;
une électrode d'ablation (120) disposée le long d'au moins la section distale du ballonnet (122) ;
dans lequel l'électrode d'ablation (120) comporte une couche externe conductrice continue sur une surface externe de la section distale du ballonnet (122) ; et
une pluralité d'électrodes de détection (146) disposées le long d'au moins la section distale du ballonnet (122), dans lequel chaque électrode de détection (146) est isolée électriquement de l'électrode d'ablation (120), et chaque électrode de détection (146) est délimitée par l'électrode d'ablation (120),
dans lequel, indépendamment d'une orientation de la section distale du ballonnet au tissu, toute ligne de contact entre la section distale du ballonnet et le tissu est sensiblement étendue en continu par l'électrode d'ablation, une ou plusieurs des électrodes de détection ou une combinaison de celles-ci.

2. Cathéter selon la revendication 1, dans lequel le long d'une surface externe du ballonnet (122), l'électrode d'ablation (120) s'étend au moins partiellement entre chaque électrode de détection (146) de la pluralité d'électrodes de détection et chacune des autres électrodes de détection (146) de la pluralité d'électrodes de détection.

3. Cathéter selon la revendication 1 ou la revendication 2, dans lequel l'électrode d'ablation (120) s'étend de manière continue entre une première électrode de détection (146) de la pluralité d'électrodes de détection et une seconde électrode de détection (146) de la pluralité d'électrodes de détection.

4. Cathéter selon l'une quelconque des revendications 1 à 3, dans lequel le volume défini par le ballonnet (122) est en communication thermique avec l'électrode d'ablation (120).

5. Cathéter selon l'une quelconque des revendications 1 à 4, dans lequel au moins la section distale du ballonnet (122) est semi-conforme.

6. Cathéter selon la revendication 5, dans lequel, en réponse à une force axiale, la section distale du ballonnet (122) est plus souple que la section proximale du ballonnet (122).

7. Cathéter selon la revendication 6, dans lequel le ballonnet (122) comporte un col (130) disposé le long de la section proximale du ballonnet (122).

8. Cathéter selon la revendication 7, dans lequel le ballonnet (122) comporte une structure de support autoextensible conçue pour dilater le ballonnet (122) d'un état plié à un état déployé.

9. Cathéter selon l'une quelconque des revendications 1 à 8, dans lequel au moins certains orifices d'irrigation (124) de la pluralité d'orifices d'irrigation s'étendent à travers l'électrode d'ablation (120).

10. Cathéter selon l'une quelconque des revendications 1 à 9, dans lequel au moins certains orifices d'irrigation (124) de la pluralité d'orifices d'irrigation sont positionnés pour diriger le fluide dans une direction proximale par rapport au ballonnet (122).

11. Cathéter selon l'une quelconque des revendications 1 à 10, dans lequel la couche externe conductrice monobloc comporte une encre conductrice disposée sur la surface externe du ballonnet (122) ou un polymère chargé avec des particules électroconductrices.

**12.** Cathéter selon l'une quelconque des revendications 1 à 11, dans lequel le ballonnet (122) est gonflable d'un état replié à un état déployé, et dans lequel, dans l'état déployé du ballonnet (122), l'électrode d'ablation (120) s'étend le long d'au moins un point de la distance du ballonnet (122) en l'absence d'une force sur une surface externe du ballonnet (122).

**13.** Cathéter selon l'une quelconque des revendications 1 à 12, comprenant en outre une pluralité de capteurs de température disposés le long du ballonnet (122), dans lequel chaque capteur de température est isolé thermiquement du fluide d'irrigation dans le volume défini par le ballonnet (122).

**14.** Cathéter selon la revendication 13, dans lequel au moins certains des capteurs de température de la pluralité de capteurs de température sont disposés le long de la section distale du ballonnet (122).

**15.** Cathéter selon l'une des revendications 1 à 14, comprenant en outre des circuits imprimés flexibles (154) disposés le long du ballonnet (122), et dans lequel chaque électrode de détection de la pluralité d'électrodes de détection (154) est formée le long d'un circuit imprimé flexible respectif (154).

**16.** Cathéter selon la revendication 1, dans lequel la couche externe conductrice recouvre plus de 90 % de la surface externe du ballonnet.

**FIG. 1**

FIG. 2

**FIG. 3**

FIG. 4

**FIG. 5**

**FIG. 6**

Step 1:
Install interducer

Step 2:
Position tip
sheath over tip

Step 3:
Push tip thru
interducer valve

Step 4:
Retract sheath

Step 5:
Advance catheter

FIG. 7

FIG. 8

FIG. 9

FIG. 10

**FIG. 11**

**FIG. 12**

FIG. 13A

FIG. 13B

FIG. 13C

**FIG. 14A**

**FIG. 14B**

FIG. 15A

FIG. 15B

FIG. 16A

FIG. 16B

**FIG. 17**

## REFERENCES CITED IN THE DESCRIPTION

**Patent documents cited in the description**

- US 62536855
- EP 3178431 A1
- US 2014081111 A1
- US 2016051321 A1